# EUROPEAN PATENT APPLICATION

(11) **EP 1 101 821 A1**
(43) Date of publication of application: **23.05.2001**
(21) Application number: 99929888.8
(22) Date of filing: 19.07.1999
(51) Int. Cl.: C12N 15/55, C12N 9/16, C12Q 1/44, C12N 1/21

(54) **NOVEL PROTEIN AND ITS DNA**

(30) Priority: 21.07.1998 JP 20496498; 16.04.1999 JP 10897499
(71) Applicant: Takeda Chemical Industries, Ltd., Osaka-shi, Osaka 541-0045 (JP)
(72) Inventor: TARUI, Naoki, Nara-shi Nara 631-0061 (JP); DOI, Takayuki, Izumi-shi Osaka 594-0013 (JP); NAKAHAMA, Kazuo, Nagaokakyo-shi Kyoto 617-0841 (JP)
(74) Representative: Caffin, Lee
(86) International application number: JP9903865
(87) International publication number: WO0005383

(57) **Abstract**

The present invention relates to a novel phosphodiesterase V, its partial peptide or a salt thereof, a DNA encoding said protein, a recombinant vector comprising said DNA, a transformant, a method for producing said protein, a screening method/a screening kit for a compound or its salt which promotes or inhibits the phosphodiesterase activity of said protein and the like.

A protein of the invention is useful as a reagent for screening for a compound or its salt, which promotes or inhibits the phosphodiesterase activity of the protein of the invention.

## Description

### Technical Field

The present invention relates to a novel protein exhibiting a phosphodiesterase activity (novel human phosphodiesterase V) and its DNA.

### Background Art

A phosphodiesterase is an enzyme which hydrolyzes cyclic adenosine 3',5'-monophosphate (cAMP) and cyclic guanosine 3',5'-monophosphate (cGMP) which are intracellular signal transmitters, and involved in the regulation of the expression of a function in various cells. Among the seven isozymes of the phosphodiesterase which are now known to be present, type-V is a enzyme exerting its effect specifically on the cGMP.

The cGMP is involved in a relaxation of a smooth muscle, an inhibition of the proliferation of a smooth muscle cell, an inhibition of an adhesion of a blood cell onto an endothelium, an inhibition of a platelet aggregation and the like whose levels are controlled by a phosphodiesterase V, which is investigated increasingly in these days. However, the report of a human phosphodiesterase (Kodera et al. Biochemistry, 69, 637 (1997)) was the only report so far with regard to a human phosphodiesterase V DNA.

### Disclosure of Invention

A discovery of a novel human protein of a phosphodiesterase V which is an enzyme having an effect specifically on a cGMP, an antibody against said protein, a screening method for an inhibitor employing said protein and the like, may enable a development of a novel pharmaceutical useful in treating various diseases involving a phosphodiesterase V (for example, a circulatory disease (angina of effort or resting (coronary contractile angina or hetero-angina), hypertension, congestive heart insufficiency, pulmonary hypertension, atherosclerosis, reduced vascular patency, stenosis, peripheral vascular disease, cerebral apoplexy and the like), occlusive pulmonary disease (chronic asthma, bronchitis and the like), allergic disease (allergic asthma, allergic rhinitis, hives and the like), a disease characterized by the motility of a digestive tract (irritable bowel syndrome and the like), glomerular disease, tubular interstitial disease, renal failure, diabetic complication, glaucoma, erectile dysfunction in male mammalian animals including human, female dysfunction in female mammalian animals including human, premature birth, dysmenorrhea and the like).

While no phosphodiesterases other than a phosphodiesterase IV have been expressed successfully using E.coli, the present invention, which was successful in expressing a phosphodiesterase V using E.coli, enables an industrial production of a phoshodiesterase V.

We made an effort to solve the problems described above, cloned successfully a cDNA having a novel base sequence from a human lung-derived cDNA library, and then discovered that a protein encoded by this DNA is a phosphodiesterase.

Based on these findings, we made a further effort to establish the present invention.

Thus, the invention relates to:
(1) a protein having an amino acid sequence identical or substantially identical to the amino acid sequence represented by SEQ. ID. NO:7 or its salt,
(2) the protein according to above (1) wherein the substantially identical amino acid sequence is the amino acid sequence represented by SEQ. ID. NO:1,
(3) the protein according to above (1) which is encoded by a cDNA possessed by an E.coli plasmid pPDE51 designated by Deposition No. FERM BP-6417,
(4) the protein according to above (1) having a phosphodiesterase activity,
(5) a partial peptide of the protein according to above (1) comprising the first to the tenth amino acid sequence from the N-terminal of the amino acid sequence represented by SEQ. ID. NO:7 or its salt,
(6) a DNA comprising a DNA having a base sequence encoding the protein according to above (1) or the partial peptide according to above (5),
(7) the DNA according to above (6) having the base sequence represented by SEQ. ID. NO:9,
(8) a recombinant vector comprising the DNA according to above (6),
(9) the recombinant vector according to above (8) which is an expression vector in E.coli,
(10) a transformant possessing the recombinant vector according to above (8),
(11) the transformant according to above (10) whose host cell is E.coli,
(12) a method for producing the protein according to above (1) or its salt comprising incubating the transformant according to above (10) to produce and accumulate the protein according to above (1) followed by collecting this protein,
(13) an antibody against the protein according to above (1), the partial peptide according to above (5) or its salt,
(14) a method for screening for a compound or its salt which promotes or inhibits the phosphodiesterase activity of the protein according to above (1), the partial peptide according to above (5) or its salts comprising using the protein according to above (1), the partial peptide according to above (5) or its salt, and
(15) a kit for screening for a compound or its salt which promotes or inhibits the phosphodiesterase activity of the protein according to above (1), the partial peptide according to above (5) or its salt, comprising using the protein according to above (1), the partial peptide according to above (5) or its salt.

Furthermore, the invention relates to:
(16) a compound obtained by using the screening method according to above (14) or the screening kit according to above (15) or its salt,
(17) a DNA comprising a DNA having a base sequence capable of being hybridized under a high stringent condition with the base sequence represented by SEQ.ID.NO:9,
(18) a recombinant vector comprising a DNA according to above (17),
(19) a transformant possessing the recombinant vector according to above (18),
(20) a method for producing a protein or its salt encoded by the DNA according to above (17) comprising incubating the transformant according to above (19) to produce and accumulate a protein followed by collecting this protein,
(21) a protein or its salt produced by the method for producing according to above (20),
(22) the method for screening according to above (11) wherein the protease (phosphodiesterase) activities of the protein according to above (1), the partial peptide according to above (5) or its salt (i) when bringing the protein according to above (1), the partial peptide according to above (5) or its salt into contact with a substrate and (ii) when bringing the protein according to above (1), the partial peptide according to above (5) or its salt into contact with the substrate and a test compound are determined and compared.

### Brief description of Drawings

Figure 1 shows the amino acid sequence of a human lung-derived phosphodiesterase V according to the invention (continued to Figure 2).
Figure 2 shows the amino acid sequence of a human lung-derived phosphodiesterase V according to the invention (continued from Figure 1).

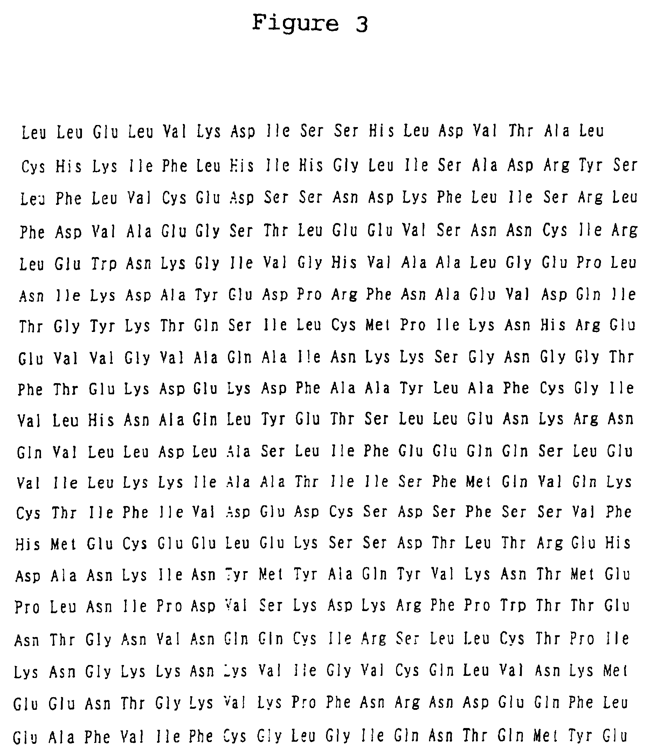

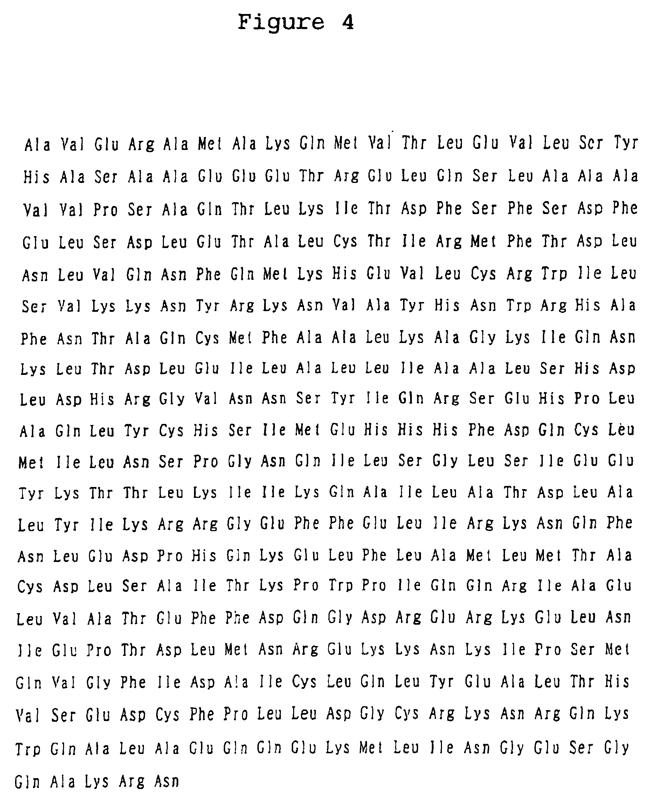

Figure 3 shows the amino acid sequence of a partial peptide of a human lung-derived phosphodiesterase V according to the invention. This is the amino acid sequence resulted from the deletion of 109 amino acids starting from the N-terminal of the amino acid sequence shown in Figure 1 and Figure 2 (continued to Figure 4).
Figure 4 shows the amino acid sequence of a partial peptide of a human lung-derived phosphodiesterase V according to the invention. This is the amino acid sequence resulted from the deletion of 109 amino acids starting from the N-terminal of the amino acid sequence shown in Figure 1 and Figure 2 (continued from Figure 3).
Figure 5 shows the sequence of a DNA containing a DNA encoding a human lung-derived phosphodiesterase V according to the invention (continued to Figure 6).
Figure 6 shows the sequence of a DNA containing a DNA encoding a human lung-derived phosphodiesterase V according to the invention (continued from Figure 5).

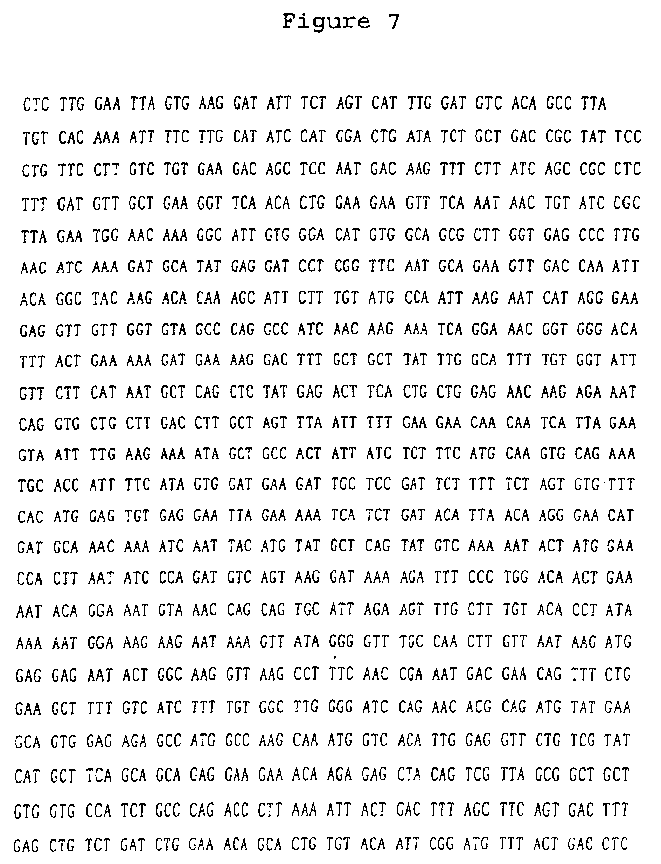

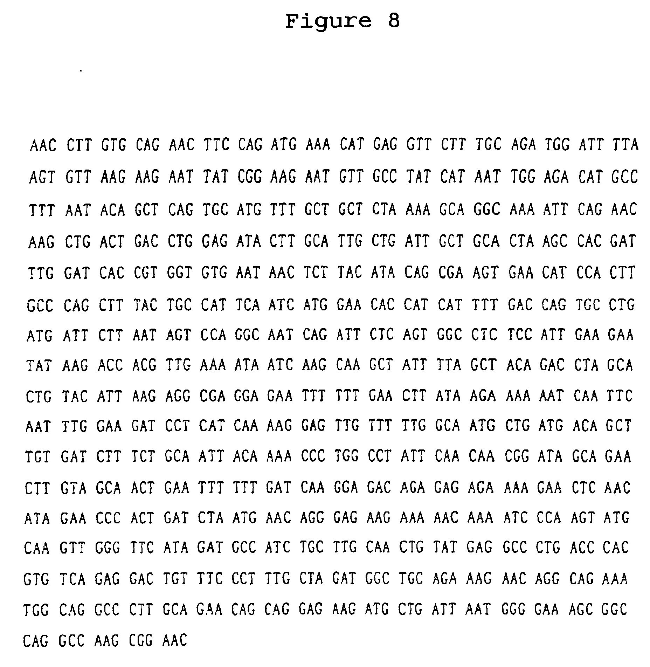

Figure 7 shows the sequence of a DNA encoding the partial peptide shown in Figure 3 and Figure 4 (continued to Figure 8).
Figure 8 shows the sequence of a DNA encoding the partial peptide shown in Figure 3 and Figure 4 (continued from Figure 7).

### Best Mode for Carrying Out the Invention

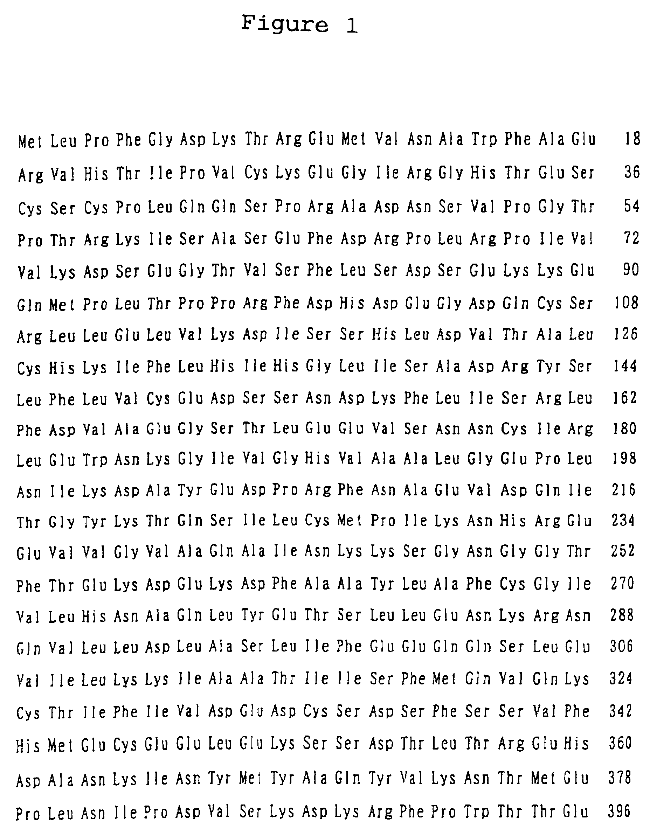

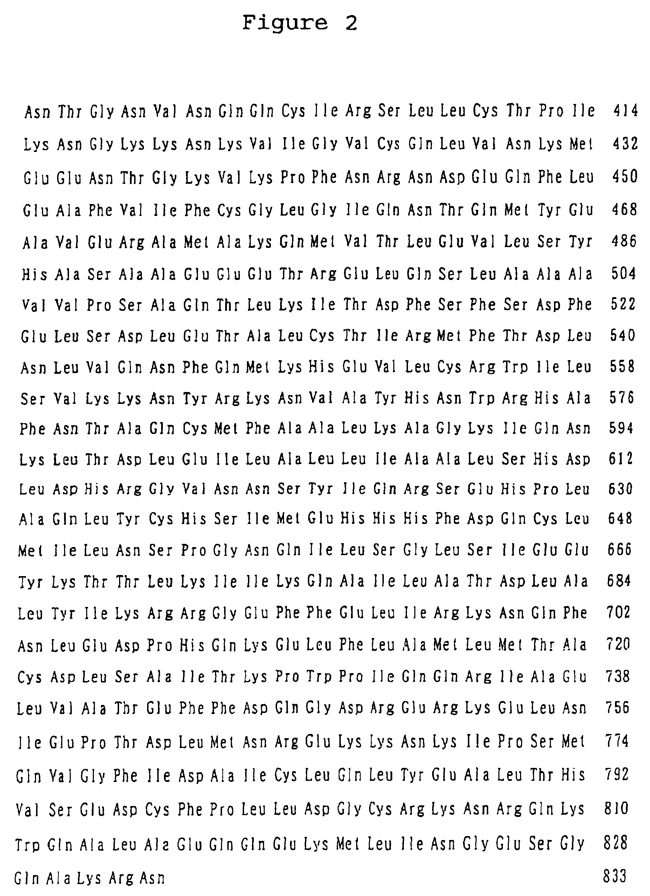

A protein having an amino acid sequence identical or substantially identical to the amino acid sequence represented by SEQ. ID. NO:7 (Figure 1 and Figure 2) (hereinafter referred to as a protein of the invention) may be a protein derived from a cell (for example, hepatocyte, splenic cell, neurocyte, gliacyte, pancreatic β cell, myelocyte, mesangial cell, Langerhans cell, epidermic cell, epithelial cell, endothelial cell, fibroblast, fibrocyte, myocyte, fat cell, immunocyte (e.g., macrophage, T cell, B, cell, natural killer cell, mast cell, neutrophile, basophile, eosinophile, monocyte), megakaryocyte, synovial cell, chondrocyte, osteocyte, osteoblast, osteoclast, mammary gland cell, hepatocyte or interstitial cell, or a precursor cell therefor, stem cell or cancer cell and the like) of a human or a warm-blooded animal (for example guinea-pig, rat, mouse, chicken, rabbit, swine, sheep, cattle, monkey and the like) or any tissue containing the cells listed above such as brain, brain parts (e.g., olfactory bulb, tonsillar nucleus, cerebral basal bulb, hippocampus, thalamus, hypothalamus, cerebral cortex, medulla oblongata, cerebellum), penis, spinal cord, pituitary gland, stomach, pancreas, kidney, liver, gonad, thyroid gland, gallbladder, bone marrow, adrenal gland, skin, muscle, lung, digestive tract (e.g., large intestine, small intestine), blood vessel, heart, thymus, spleen, submandibular gland, peripheral blood, prostate, testis, ovary, placenta, uterus, bone, joint, skeletal muscle and the like, or a blood cell or a cultured cell line thereof (e.g., MEL, M1, CTLL-2, HT-2, WEHI-3, HL-60, JOSK-1, K562, ML-1, MOLT-3, MOLT-4, MOLT-10, CCRF-CEM, TALL-1, Jurkat, CCRT-HSB-2, KE-37, SKW-3, HUT-78, HUT-102, H9, U937, THP-1, HEL, JK-1, CMK, KO-812, MEG-01 and the like) as well as a synthetic protein.

An amino acid sequence substantially identical to the amino acid sequence represented by SEQ. ID. NO:7 may for example be an amino acid comprising the first to the tenth amino acid sequence from the N-terminal of the amino acid sequence represented by SEQ. ID. NO:7 and having about 92 % or more, preferably about 95 % or more, more preferably about 98 % or more of the homology with the amino acid sequence represented by SEQ. ID. NO:7.

A protein encoded by a cDNA possessed by an E.coli plasmid pPDE51 designated by Deposition No. FERM BP-6417 described below may also be exemplified as a protein according to the invention.

An amino acid sequence substantially identical to the amino acid sequence represented by SEQ. ID. NO:7 includes an amino acid sequence comprising the first to the tenth amino acid sequence from the N-terminal of the amino acid sequence represented by SEQ. ID. NO:7 and having the 11th to the 15th amino acid sequence and the 546th to the 833rd (more preferably the 437th to the 724th) amino acid sequence. More typically, an amino acid sequence substantially identical to the amino acid sequence represented by SEQ. ID. NO:7 includes the amino acid sequence represented by SEQ. ID. NO:1.

A protein having an amino acid sequence substantially identical to the amino acid sequence represented by SEQ. ID. NO:7 may for example be a protein having an amino acid sequence substantially identical to the amino acid sequence represented by SEQ. ID. NO:7 and having an activity substantially similar to that of the protein having the amino acid sequence represented by SEQ. ID. NO:7 (typically a phosphodiesterase activity, more preferably a phosphodiesterase V activity, most preferably, a phosphodiesterase activity specific to a protein having the amino acid sequence represented by SEQ. ID. NO:7, cGMP activity and the like).

The expression "substantially similar" means that the activity is qualitatively (e.g., physiochemically or pharmacologically). Accordingly, it is preferable that the activity is equivalent (e.g., about 0.1 to 100 times, preferably about 0.5 to 10 times, more preferably 0.5 to 2 times), while the quantitative aspects such as the magnitude of the activity and the molecular weight of the protein may be different.

The activity of a protein having the amino acid sequence represented by SEQ. ID. NO:7 (typically a phosphodiesterase activity, more preferably a phosphodiesterase V activity, most preferably, a phosphodiesterase activity specific to a protein having the amino acid sequence represented by SEQ. ID. NO:7, cGMP activity and the like) can be determined by a method known per se, or by a screening method described below.

A protein according to the invention also includes a protein comprising the first to the tenth amino acid sequence from the N-terminal of the amino acid sequence represented by SEQ. ID. NO:7 and also comprising [1] an amino acid sequence formed as a result of the deletion of one or more (preferably 1 to about 30, more preferably 1 to about 10, most preferably 1 to 5) amino acids in the amino acid sequence represented by SEQ. ID. NO:7, [2] an amino acid sequence formed as a result of the addition of one or more (preferably 1 to about 30, more preferably 1 to about 10, most preferably 1 to 5) amino acids to the amino acid sequence represented by SEQ. ID. NO:7, [3] an amino acid sequence formed as a result of the insertion of one or more (preferably 1 to about 30, more preferably 1 to about 10, most preferably 1 to 5) amino acids into the amino acid sequence represented by SEQ. ID. NO:7, [4] an amino acid sequence formed as a result of the substitution of one or more (preferably 1 to about 30, more preferably 1 to about 10, most preferably 1 to 5) amino acids in the amino acid sequence represented by SEQ ID. NO:7 with other amino acids or [5] an amino acid as a combination thereof, which is referred also as mutein,

While such insertion, deletion or substitution of the amino acids may occur in a position which is not particularly limited, such position may for example be other than the 437th to the 724th, more preferably the 546th to the 833rd amino acids sequence of the amino acid sequence represented by SEQ ID. NO:7.

A protein according to the invention has an N-terminal (amino terminal) in its left end, a C-terminal (carboxyl terminal) in the right end as ordinarily in the peptide designation. While a protein of the invention including a protein comprising the amino acid sequence represented by SEQ. ID. NO:7 as its representative usually has a carboxyl group (-COOH) or a carboxylate (-COO⁻) at its C-terminal, it may have an amide (-CONH₂) or an ester (-COOR) at its C-terminal.

R in an ester employed here may for example be a C₁₋₆ alkyl group such as methyl, ethyl, n-propyl, isopropyl and n-butyl, a C₃₋₈ cycloalkyl group such as cyclopentyl and cyclohexyl, a C₆₋₁₂ aryl group such as phenyl and α-naphthyl and a C₇₋₁₄ aralkyl including a phenyl-C₁₋₂ alkyl such as benzyl and phenethyl and an α-naphthyl-C₁₋₂ alkyl such as α-naphthylmethyl as well as a pivaloyloxymethyl group employed widely as an oral ester.

When a protein of the invention has a carboxyl group (or carboxylate) anywhere other than its C-terminal, it may also be included in the invention when such carboxyl group is amidated or esterified. The ester in such case may be an ester in the C-terminal listed above.

A protein of the invention also includes one whose amino group in the amino acid residue at the N-terminal (e.g., methionine residue) is protected by a protective group (for example, C₁₋₆ acyl group including a C₁₋₆ alkanoyl group such as formyl and acetyl groups), one whose Gln at the N-terminal formed as a result of an in vivo cleavage is converted into a pyroglutamic acid, one whose substituent on a side chain of an intramolecular amino acid (for example, -OH, -SH, amino group, imidazole group, indole group, guanidino group) is protected by a suitable protective group (for example, C₁₋₆ acyl group including a C₁₋₆ alkanoyl group such as formyl and acetyl groups), or a conjugated protein such as a glycoprotein having a saccharide chain bound thereto.

A partial peptide of a protein of the invention may for example be a partial peptide of a protein of the invention described above comprising the amino acid sequence of the first to the tenth amino acid sequence from the N-terminal of the amino acid sequence represented by SEQ. ID. NO:7 and preferably having the activity similar to that of the protein of the invention described above (typically a phosphodiesterase activity, more preferably a phosphodiesterase V activity, most preferably, a phosphodiesterase activity specific to a protein having the amino acid sequence represented by SEQ. ID. NO:7, cGMP activity and the like). For example, a peptide comprising the amino acid sequence of the first to the tenth amino acid sequence from the N-terminal of the amino acid sequence represented by SEQ. ID. NO:7 and having at least 20 or more, preferably 50 or more, more preferably 70 or more, further preferably 100 or more, most preferably 200 or more amino acid sequence among the constituent amino acid sequence of the protein of the invention and also having the activity similar to that of the protein of the invention (typically a phosphodiesterase activity, more preferably a phosphodiesterase V activity, most preferably, a phosphodiesterase activity specific to a protein having the amino acid sequence represented by SEQ. ID. NO:7. cGMP activity and the like) is employed.

More typically, a peptide comprising the amino acid sequence of the first to the 15th (more preferably the first to the tenth) amino acid sequence represented by SEQ. ID. NO:7 and having at least 20 or more, preferably 50 or more, more preferably 70 or more, further preferably 100 or more, most preferably 200 or more amino acid sequence among the constituent amino acid sequence of the protein of the invention, more specifically having an amino acid sequence represented by SEQ. ID. NO:2, and also having the activity similar to that of the protein of the invention (typically a phosphodiesterase activity, more preferably a phosphodiesterase V activity, most preferably, a phosphodiesterase activity specific to a protein having the amino acid sequence represented by SEQ. ID. NO:7, cGMP activity and the like) is employed.

While a partial peptide of the invention usually has a carboxyl group (-COOH) or a carboxylate (-COO⁻) at its C-terminal, it may have an amide (-CONH₂) or an ester (-COOR, wherein R is defined as described above) at its C-terminal similarly to a protein of the invention.

A partial peptide of the invention also includes, similarly to a protein of the invention, one whose amino group in the amino acid residue at the N-terminal (e.g., methionine residue) is protected by a protective group, one whose Gln as a result of an in vivo cleavage at the N-terminal formed is converted into a pyroglutamic acid, one whose substituent on a side chain of an intramolecular amino acid is protected by a suitable protective group, or a conjugated protein such as a glycoprotein having a saccharide chain bound thereto.

Since a partial peptide of the invention can be used as an antigen for preparing an antibody, it should not necessarily have a phosphodiesterase activity.

A salt of a protein or a partial peptide of the invention may be a salt with a physiologically acceptable acid (e.g., inorganic acid, organic acid) or with a base (e.g., alkaline metal), and a salt preferred especially is a physiologically acceptable acid addition salt. Such salt may for example be a salt with an inorganic acid (for example, hydrochloric acid, phosphoric acid, hydrobromic acid, sulfuric acid), or with an organic acid (for example, acetic acid, formic acid, propionic acid, fumaric acid, maleic acid, succinic acid, tartaric acid, citric acid, malic acid, oxalic acid, benzoic acid, methanesulfonic acid, benzenesulfonic acid).

A protein or a partial peptide of the invention or its salt may be produced by a known method for purifying a protein from a cell or a tissue of human or a warm-blooded animal described above, or may be produced by incubating a transformant comprising a DNA encoding a protein as described below. Alternatively, a peptide synthesis described below may also be employed.

When a tissue or a cell of human or a mammalian animal is used as a starting material, it is homogenized and extracted, for example, with an acid to obtain an extract, which is then purified by a combination of chromatographic methods such as a reverse phase chromatography, an ion exchange chromatography and the like.

A protein or a partial peptide of the invention or its salt or amide may usually be synthesized using a commercial protein synthesis resin. Such resin may for example be a chloromethyl resin, a hydroxymethyl resin, a benzhydrylamine resin, an aminomethyl resin, a 4-benzyloxybenzyl alcohol resin, a 4-methylbenzhydrylamine resin, a PAM resin, a 4-hydroxymethylmethylphenylacetamidemethyl resin, a polyacrylamide resin, a 4-(2',4'-dimethoxyphenylhydroxymethyl)phenoxy resin, a 4-(2',4'-dimethoxyphenyl-Fmoc aminoethyl)phenoxy resin and the like. Using such resin, an amino acid whose α-amino group and side chain functionalities are protected suitably is condensed on the resin according to any condensation method known per se in the order of the sequence of an intended protein. At the end of the reaction, a protein is isolated from the resin with being deprotected simultaneously, and then subjected to an intramolecular disulfide bond forming reaction in a highly diluted solution to obtain an intended protein or its amide.

While the condensation of a protected amino acid described above may be effected using various activating reagents which can be employed for synthesizing a protein, a carbodiimide is employed preferably. Such carbodiimide may be DCC, N,N'-diisopropylcarbodiimide, N-ethyl-N'-(3-dimethylaminopropyl)carbodiimide and the like. For an activation using any of those listed above, a protected amino acid may be added directly to a resin together with a racemization-inhibiting auxiliary agent (for example, HOBt, HOOBt), or a protected amino acid may previously be activated as a symmetric acid anhydride or an HOBt ester or an HOOBt ester and then added to a resin.

A solvent used in an activation of a protected amino acid or in a condensation with a resin may be one selected from the solvents known to be useful in a protein condensation reaction. Those employed may for example be an acid amide such as N,N-dimethylformamide, N,N-dimethylacetamide and N-methylpyrrolidone, a halogenated hydrcarbon such as methylene chloride and chloroform, an alcohol such as trifluoroethanol, a sulfoxide such as dimethylsulfoxide, an ether such as pyridine, dioxane and tetrahydrofuran, a nitrile such as acetonitrile and propionitrile, an ester such as methyl acetate and ethyl acetate, or a mixture thereof. The reaction temperature may appropriately be selected from the range known to be useful in a protein binding reaction, and may usually range from -20°C to 50°C. An activated amino acid derivative is employed usually In excess of 1.5 to 4 times. When a ninhydrin reaction test revealed an insufficient condensation, the condensation is repeated without any deprotection to achieve a sufficient condensation. When a repetitive condensation is still not successful in achieving a sufficient condensation, acetic anhydride or acetylimidazole may be employed for acetylating an unreacted amino acid to avoid any effect on a subsequent reaction.

A protective group for an amino group of a starting material may for example be Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulfenyl, diphenylphosphonothioyl, Fmoc and the like.

A carboxyl group can be protected for example by an alkylesterification (for example a straight, branched or cyclic alkylesterification employing methyl, ethyl, propyl, butyl, t-butyl, cyclopentyl, cyclohexyl, cycloheptyl, cyclooctyl, 2-adamantyl and the like), an aralkylesterification (for example benzylesterification, 4-nitrobenzylesterification, 4-methoxybenzylesterification, 4-chlorbenzylesterification, benzhydrylesterification), phenacylesterification and can also be protected as benzyloxycarbonylhydrazide, t-butoxycarbonylhydrazide, tritylhydrazide and the like.

The hydroxyl group of serine can be protected for example by an esterification or an etherification. A group suitable in such esterification may for example be a lower (C₁₋₆) alkanoyl group, an aroyl group such as a benzoyl group, or a group derivatized from carbonic acid such as a benzyloxycarbonyl group and an ethoxycarbonyl group. A group suitable in such etherification may for example be a benzyl, tetrahydropyranyl and t-butyl groups.

A protective group for a phenolic hydroxyl group of tyrosine may for example be Bzl, C₁₂-Bzl, 2-nitrobenzyl, Br-Z, t-butyl and the like.

A protective group for imidazole of histidine may for example be Tos, 4-methoxy-2,3,6-trimethylbenzenesulfonyl, DNP, benzyloxymethyl, Bum, Boc, Trt, Fmoc and the like.

A starting substance whose carboxyl group is activated may for example be a corresponding acid anhydride, an azide, an activated ester [ester with alcohol (for example pentachlorophenol, 2,4,5-trichlorophenol, 2,4-dinitrophenol, cyanomethyl alcohol, p-nitrophenol, HONB, N-hydroxysuccinimide, N-hydroxyphthalimide, HOBt)]. A starting substance whose amino group is activated may for example be a corresponding phosphoryl amide.

A method for a deprotection (cleavage) may be a catalytic hydrogenation under a hydrogen flow in the presence of a catalyst such as Pd-black or Pd-C, a treatment with an acid such as anhydrous hydrofluoric acid, methanesulfonic acid, trifluoromethanesulfonic acid, trifluoroacetic acid or a mixture thereof, a treatment with a base such as diisopropylethylamine, triethylamine, piperidine, piperazine and the like, as well as a reduction with sodium in a liquid ammonia. A cleavage employing an acid treatment described above is performed usually at a temperature of -20°C to 40°C, and such acid treatment is effected advantageously by adding a cation scavenger such as anisol, phenol, thioanisol, m-cresol, p-cresol, dimethylsulfide, 1,4-butanedithiol, 1,2-ethanedithiol and the like. A 2,4-dinitrophenyl group employed as an imidazole protective group for histidine is deprotected by a thiophenol treatment, while a formyl group employed as an indole protective group of tryptophan is removed by a deprotection using an acid treatment in the presence of 1,2-ethanedithiol and 1,4-butandithiol as described above and also by a treatment with an alkali such as a dilute solution of sodium hydroxide and a dilute ammonia.

A protection of a functional group which should no be involved in a reaction, a protective group therefor, a deprotection of such protective group and an activation of a functional group involved in a reaction may appropriately be selected from the groups and the methods known per se.

In an alternative method for obtaining an amide of a protein, the α-carboxyl group of the amino acid at the carboxy terminal may for example be protected as being amidated, and then a peptide chain (protein) is elongated to a desired length in the direction of its amino group, and then a protein from which only the protection group of the α-amino group at the N-terminal of the peptide and a protein from which only the protection group of the carboxyl group at the C-terminal of are produced, and the both proteins are condensed in a solvent mixture described above. Such condensation is detailed above. After purifying a protected protein obtained by the condensation, all protective groups are removed by the methods described above to yield a desired crude protein. This crude protein can be purified by any of the known purification means and a target fraction is lyophilized to obtain an amide of the desired protein.

For obtaining an ester of a protein, the α-carboxyl group of the amino acid at the carboxy terminal may for example be condensed with a desired alcohol to form an amino acid ester, which is converted into an ester of a desired protein similarly to an amide of a protein.

A partial peptide of the invention or its salt can be produced according to a peptide synthesis method known per se or by cleaving a protein of the invention with an appropriate peptidase. Such peptide synthesis method may be a solid phase synthesis or a liquid phase synthesis. Thus, a partial peptide or an amino acid capable of constituting a partial peptide of the invention is condensed with the remainder moiety and then a protective group, if any, of the product is removed to obtain an intended peptide. Examples of the known condensation methods and deprotection methods are described in References [1] to [5] shown below.
[1] M.Bodanszky and M.A. Ondetti, Peptide Synthesis, Interscience Publishers, New York (1966);
[2] Schroeder and Luebke, The Peptide, Academic Press, New York (1965);
[3] N.Izumi et al, Basics and experiments of peptide synthesis, Maruzen (1975);
[4] H.Yajima et al, Biochemisty experiment 1, Protein chemistry IV, 205 (1977);
[5] H.Yajima (supervision), Pharmaceutical development II, Vol.14, Peptide synthesis, Hirokawa Shoten.

After the reaction, a partial peptide of the invention can be isolated and purified by a combination if a solvent extraction, a distillation, a column chromatography, a liquid chromatography, a recrystallization and the like. When the partial peptide thus obtained is in a free form then it can be converted into a suitable salt by a known method or its modification, and when the product is a salt then it can be converted into a free form or other salts by a known method or its modification.

A DNA encoding a protein of the invention may be any of those described above which comprises a base sequence encoding the protein of the invention. It may also be a genome DNA, a genome DNA library, a cDNA derived from a cell or a tissue described above, a cDNA library derived from a cell or a tissue described above and a synthetic DNA.

A vector employed to obtain a library may be a bacteriophage, a plasmid, a cosmid, a phagimid and the like. A total RNA or a mRNA fraction prepared from a cell or a tissue described above may also be used directly in an amplification by a reverse transcriptase polymerase chain reaction (hereinafter abbreviated as RT-PCR method).

A DNA encoding a protein of the invention may for example be [1] a DNA comprising the base sequence represented by SEQ. ID. NO:9 or a DNA having a base sequence capable of being hybridized under a high stringent condition with the base sequence represented by SEQ. ID. NO:9 and encoding a protein having an activity substantially similar to that of the protein of the invention (e.g., a phosphodiesterase activity, more preferably a phosphodiesterase V activity, most preferably, a phosphodiesterase activity specific to a protein having the amino acid sequence represented by SEQ. ID. NO:7, cGMP activity and the like).

A DNA capable of being hybridized under a high stringent condition with the base sequence represented by SEQ. ID. NO:9 may for example be a DNA comprising the 30 base sequence starting from the 5' terminal of the base sequence represented by SEQ. ID. NO:9, and having about 92 % or more, preferably about 95 % or more, most preferably about 98 % or more of the homology with the base sequence represented by SEQ. ID. NO:9.

A hybridization can be performed by a method known per se or its modification, for example, a method described in Molecular Cloning, 2nd, J.Sambrook et al., Cold Spring Harbor Lab. Press (1989). When a commercial library is employed, an attached instruction may be followed. More preferably, a high stringent condition is employed.

A high stringent condition may for example be one employing a sodium concentration of about 19 to 40 mM, preferably about 19 to 20 mM and a temperature of about 50 to 70°C, preferably about 60 to 65°C. A sodium concentration of about 19 mM combined with a temperature of about 65°C is most preferred.

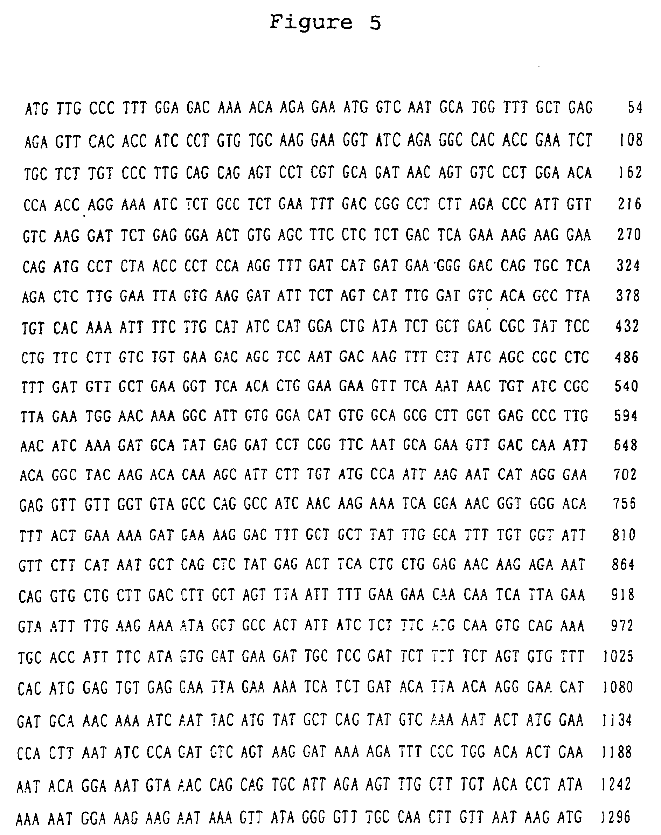

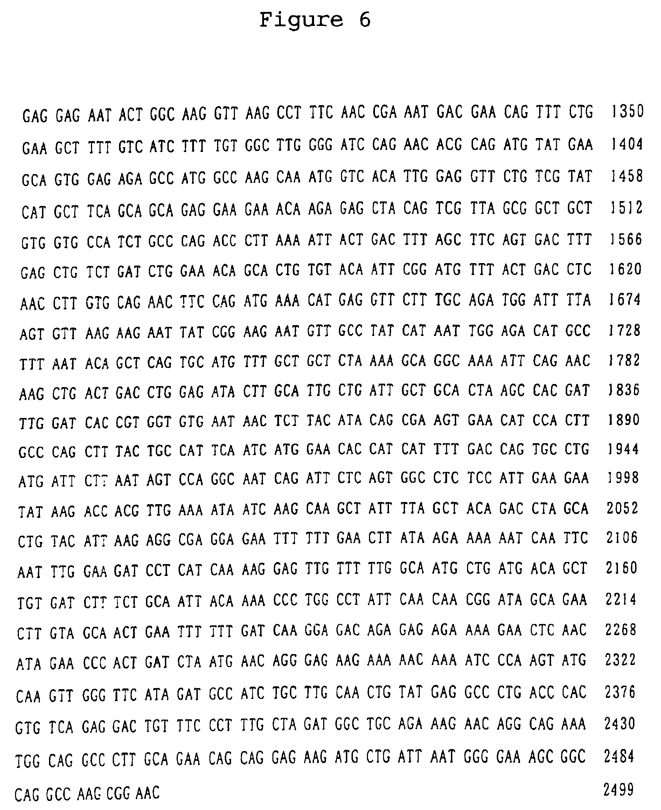

More typically, a DNA encoding a protein having the amino acid sequence represented by SEQ. ID. NO:7 may be a DNA having the base sequence represented by SEQ. ID. NO:9 (the 1st to the 2499th base sequence in Figures 5 and 6). A DNA encoding a protein having the amino acid sequence represented by SEQ. ID. NO:1 may be a DNA having the base sequence represented by SEQ. ID. NO:3.

A DNA encoding a partial peptide of the invention may be any of those described above which comprises a base sequence encoding the partial peptide of the invention. It may also be a genome DNA, a genome DNA library, a cDNA derived from a cell or a tissue described above, a cDNA library derived from a cell or a tissue described above and a synthetic DNA.

A DNA encoding a partial peptide of the invention may for example be a DNA comprising the 30 base sequence starting from the 5' terminal of the base sequence represented by SEQ. ID. NO:9, and having [1] a partial base sequence of a DNA having the base sequence represented by SEQ. ID. NO:9 or having [2] a base sequence capable of being hybridized under a high stringent condition with a DNA having the base sequence represented by SEQ. ID. NO:9 and having a partial base sequence of a DNA encoding a protein having the amino acid sequence represented by SEQ. ID. NO:7 (typically a phosphodiesterase activity, more preferably a phosphodiesterase V activity, most preferably, a phosphodiesterase activity specific to a protein having the amino acid sequence represented by SEQ. ID. NO:7, cGMP activity and the like). More typically, a DNA comprising the 30 base sequence starting from the 5' terminal of the base sequence represented by SEQ. ID. NO:9 and having the base sequence represented by SEQ. ID. NO:4 is exemplified.

In a method for cloning aDNA which completely encodes an inventive protein or partial peptide (hereinafter they are referred to together as a protein of the invention in the following description with regard to the cloning and the expression of a DNA encoding them), a synthetic DNA primer having a partial base sequence of the protein of the invention is employed for an amplification by a PCR method, or a selection is accomplished by means of a hybridization of a DNA labeled with a DNA fragment encoding a part or all of the protein of the invention or a synthetic DNA with a DNA integrated into a suitable vector. A hybridization can be performed for example by a method described in Molecular Cloning, 2nd, J.Sambrook et al., Cold Spring Harbor Lab. Press (1989). When a commercial library is employed, an attached instruction may be followed.

The conversion of the base sequence of a DNA can be performed by a method known per se such as Gupped duplex method or Kunkel method or its modification using a known kit, such as MutantTM-G (TAKARA SHUZO CO., LTD.) or MutantTM-K (TAKARA SHUZO CO., LTD.).

A DNA encoding a cloned protein can be used directly or after a digestion with a restriction enzyme or an addition of a linker if desired. Such DNA may have an ATG as a translation initiation codon at its 5' terminal and a TAA, TGA or TAG as a translation termination codon at its 3' terminal. Such translation initiation or termination codon may be added using an appropriate synthetic DNA adapter.

An expression vector for a protein of the invention can be prepared for example by (i) cutting a desired DNA fragment out of a DNA encoding the protein of the invention, and (ii) ligating said DNA fragment to the downstream of a promoter of a suitable expression vector.

Such vector may for example be an E.coli-derived plasmid (e.g., pBR322, pBR325, pUC12, pUC13, pPDE51), a B.subtilis-derived plasmid (e.g., pUB110, pTP5, pC194), an yeast-derived plasmid (e.g., pSH19, pSH15), a bacteriophage such as λ phage, an animal virus such as retrovirus, vaccinia virus, vaculovirus and the like, as well as pA1-11, pXT1, pRc/CMV, pRC/RSV, pcDNAI/Neo and the like.

For an industrial expression of a large amount of a protein of the invention, it is preferred to use a transformant whose host is E.coli, and a vector employed preferably is an E.coli-derived plasmid (e.g., pBR322, pBR325, pUC12, pUC13, pPDE51).

A promoter employed in the invention may be any promoter which is appropriate correspondingly to a host employed for expressing a gene. For example, when an animal cell is employed as a host cell, those exemplified are SRα promoter, SV40 promoter, LTR promoter, CMV promoter, HSV-TK promoter and the like.

Among those listed above, CMV (cytomegalovirus) promoter and SRα promoter are employed preferably. Those preferred for an Escherichia microorganism as a host cell are trp promoter, lac promoter, recA promoter. λPL promoter, lpp promoter, T7 promoter and the like, those preferred for a Bacillus microorganism as a host cell are SPO1 promoter, SPO2 promoter, penP promoter and the like, those preferred for an yeast as a host cell are PHO5 promoter, PGK promoter, GAP promoter, ADH promoter and the like. Those preferred for an insect cell as a host cell are polyhedrin promoter, p10 promoter and the like.

In addition to those described above, an expression vector containing, if desired, an enhancer, a splicing signal, a polyA addition signal, a selection marker, an SV 40 replication origin (hereinafter sometimes abbreviated as SV40ori) and the like may also be employed. A selection marker may for example be a dihydrofolic acid reductase (hereinafter sometimes abbreviated as dhfr) gene [methotrexate (MTX) resistant], an ampicillin resistant gene (hereinafter sometimes abbreviated as Amp^{r}), a neomycin resistant gene (hereinafter sometimes abbreviated as Neo^{r}, G418 resistant) and the like. Especially when a dhfr gene is employed as a selection marker using a dhfr gene-defect Chinese hamster cell, an intended gene can be selected using a thymidine-free medium.

A signal sequence suitable for a host cell may also be added if necessary to the N-terminal of a protein of the invention.
Those preferred for an Escherichia microorganism as a host cell are Pho A·signal sequence, Omp A·signal sequence and the like, those preferred for a Bacillus microorganism as a host cell are α-amylase·signal sequence, subtilicin·signal sequence and the like, those preferred for an yeast as a host cell are MFα· signal sequence, SUC2·signal sequence and the like, and those preferred for an animal cell as a host cell are insulin·signal sequence, α-interferon·signal sequence, an antibody molecule·signal sequence and the like.

Using a vector comprising a DNA encoding a protein of the invention thus constructed, a transformant can be prepared.

A host cell may for example be an Escherichia microorganism, a Bacillus microorganism, an yeast, an insect cell, an insect, an animal cell and the like. For an industrial expression of a large amount of a protein of the invention, it is preferred to use an Escherichia microorganism as a host cell.

Such Escherichia microorganism may for example be Eshcerichia coil K12·DH1 (Proc. Nat. Acad. Sci. USA, Vol.60, 160 (1968)), JM103 (Nucleic Acids Research, Vol.9, 309 (1981), JA221 (Journal of Molecular Biology), (Genetics, Vol.39, 440 (1954), BL21 (FUNAKOSHI) and the like.

A Bacillus microorganism may for example be Bacillus subtilis MI114 (Gene, Vol.24, 255 (1983)), 207-21 (Journal of Biochemistry, Vol.95, 87 (1984)) and the like.

An yeast may for example be Saccharomyces cerevisiae AH22, AH22R⁻, NA87-11A, DKD-5D, 20B-12, Schizosaccharomyces pombe NCYC1913, NCYC2036, Pichia pastoris KM71 and the like.

An insect cell, when a virus is AcNPV, may for example be an armyworm-derived cultured cell line, (Spodoptera frugiperda cell; Sf cell), a Trichoplusia ni mesenteron-derived MG1 cell, Trichoplusia ni egg-derived High Five™ cell, Mamestra brassicae-derived cell, Estigmena acrea-derived cell and the like. A cell when a virus is BmNPV may for example be a silkworm-derived cultured cell line (Bombyx mori N cell; BmN cell) and the lie. Such Sf cell may for example be an Sf9 cell (ATCC CRL1711), an Sf21 cell (for both, see Vaughn, J.L. et al., In Vivo, 13, 213-217 (1977)) and the like.

An insect may for example be a larva of a silkworm (Maeda et al., Nature, Vol.315, 592 (1985)).

An animal cell may for example be a simian cell COS-7, V ero, a Chinese hamster cell CHO (hereinafter abbreviated as CHO cell), a dhfr gene-defect Chinese hamster cell CHO (hereinafter abbreviated as CHO(dhfr⁻) cell), a mouse L cell, a mouse AtT-20, a mouse myeloma cell, a rat GH3, a human FL cell and the like.

An Escherichia microorganism can be transformed for example by a method described in Proc. Natl. Acad. Sci. USA, Vol.69, 2110 (1972), or Gene, Vol.17, 107 (1982).

A Bacillus microorganism can be transformed for example by a method described in Molecular & General Genetics, Vol.168, 111 (1979).

An yeast can be transformed for example by a method described in Methods in Enzymology, Vol.194, 182-187 (1991), Proc. Natl. Acad. Sci. USA. Vol.75, 1929 (1978) and the like.

An insect cell or an insect can be transformed for example by a method described in Bio/Technology, 6, 47-55 (1988).

An animal cell can be transformed for example by a method described in CELL ENGINEERING EXTRA ISSUE No.8, NEW CELL ENGINEERING PROTOCOL, 263-267 (1995) (SHUJUNSHA), Virology, Vl.52, 456 (1973) and the like.

As described above, a transformant which had been transformed with an expression vector comprising a DNA encoding a protein can be obtained.

When incubating a transformant whose host cell is an Escherichia microorganism or a Bacillus microorganism, a suitable culture medium employed is a liquid medium which may contain substances required for the growth of the relevant transformant such as carbon sources, nitrogen sources, inorganic substances and the like. A carbon source may for example be glucose, dextrin, soluble starch, sucrose and the like, and a nitrogen source may for example be an inorganic or organic material such as an ammonium salt, a nitrate, corn steep liquor, peptone, casein, meat extract, soybean bran, potato extract and the like, and an inorganic substance may for example be calcium chloride, sodium dihydrogen phosphate, magnesium chloride and the like. An yeast, a vitamin and a growth promoting factor may also be added. The pH of a medium is preferably about 5 to 8.

A preferred culture medium for incubating an Escherichia microorganism may for example a M9 medium containing glucose and casamino acid (Miller, Journal of Experiments in Molecular Genetics, 431-433, Cold Spring Harbor Laboratory, New York (1972)). If necessary, a medium may contain an agent for facilitating the action of a promoter such as 3β-indolylacrylic acid.
When an Escherichia microorganism is employed as a host cell, the incubation is performed usually at 15 to 43°C for about 3 to 24 hours, if necessary with an aeration or a stirring.

When a Bacillus microorganism is employed as a host cell, the incubation is performed usually at 30 to 40°C for about 6 to 24 hours, if necessary with an aeration or a stirring.

When incubating a transformant whose host cell is an yeast, a suitable culture medium employed may for example be a Burkholder minimum medium (Bostian, K.L. et al., Proc. Natl. Acad. Sci. USA, Vol.77, 4505 (1980), and a 0.5 % casamino acid-supplemented SD medium (Bitter, G.A. et al., Proc. Natl. Acad. Sci. USA, Vol.81, 5330 (1984). The pH of a culture medium is adjusted preferably at about 5 to 8. The incubation is performed usually at 20 to 35°C for about 24 to 72 hours, if necessary with an aeration or a stirring.

When incubating a transformant whose host cell is an insect cell or an insect, a suitable culture medium employed may for example be a Grace's Insect Medium (Grace, T.C.C., Nature, 195, 788 (1962) supplemented appropriately, for example, with an inactivated 10 % bovine serum. The pH of a culture medium is adjusted preferably at about 6.2 to 6.4. The incubation is performed usually at 27°C for about 3 to 5 days, if necessary with an aeration or a stirring.

When incubating a transformant whose host cell is an animal cell, a suitable culture medium employed may for example be a MEM medium supplemented with about 5 to 20 % fetal bovine serum (Science, Vol.122, 501 (1952), a DMEM medium (Virology, Vol.8, 396 (1959), an RPMI1640 medium (The Journal of the American Medical Association, Vol.199, 519 (1967), a 199 medium (Proceedings of the Society for the Biological Medicine, Vol.73, 1 (1950), and the like. The pH of a culture medium is preferably about 6 to 8. The incubation is performed usually at 30 to 40°C for about 15 to 60 hours, if necessary with an aeration or a stirring.

As described above, a protein according to the invention can be produced in a cell membrane of a transformant.

In order to separate and purify a protein of the invention from a cell culture described above, a method described below may for example be employed.

For extracting a protein of the invention from a cultured microorganism or a cultured cell, the cell is collected by a known method after an incubation and suspended in a suitable buffer solution, which is subjected to an ultrasonication, a treatment with lysozyme and/or a freezing and thawing cycle to destruct the cell followed by a centrifugation or a filtration to obtain a crude extract of the protein. The buffer solution may contain a protein modifier such as urea or guanidine hydrochloride or a surfactant such as Triton X-100™. When a protein is secreted into a culture medium, then a cell and a supernatant are separated by a method known per se after completing the incubation to collect the supernatant.

The purification of a protein contained in a culture supernatant or an extract thus obtained may be performed by an appropriate combination of separation and purification methods known per se. Such known separation and purification methods are a method utilizing a solubility such as a salting out or a solvent precipitation, a method mainly utilizing the difference in the molecular weight such as a dialysis, a ultrafiltration, a gel filtration and an SDS-polyacrylamide gel electrophoresis, a method utilizing the difference in the electric charge such as an ion exchange chromatography, a method utilizing the difference in the hydrophobicity such as a reverse phase high pressure liquid chromatography, a method utilizing the difference in the isoelectric point such as an isoelectric focusing and the like.

When a protein thus obtained is in a free form then it can be converted into a salt by a method known per se or its modification, and, on the contrary, when it is obtained as a salt then it can be converted into a free form or another salt by a method known per se or its modification.

It is also possible that a protein produced by a recombinant is treated with a suitable protein-modifying enzyme before or after a purification to achieve a desired modification or a partial removal of a polypeptide. Such protein-modifying enzyme may for example be trypsin, chymotrypsin, arginylendopeptidase, protein kinase, glycosidase and the like.

A protein of the invention or its salt thus produced can be examined for its presence or activity for example by a labeled ligand binding test or an enzyme immunoassay employing a specific antibody.

An antibody against a protein or a partial peptide of the invention or its salt may be either a polyclonal antibody or a monoclonal antibody provided that it can recognize such protein or partial peptide of the invention or its salt.

An antibody against a protein or a partial peptide of the invention or its salt (hereinafter referred to together as a protein of the invention for convenience) can be produced using the protein of the invention as an antigen by a known method for producing an antibody or antiserum.

### [Monoclonal antibody preparation]

### (a) Preparation of monoclonal antibody-producing cell

A protein of the invention can be administered as it is or in combination with a carrier or diluent to a site where the antibody can be produced in response to the administration to a warm-blooded animal. The administration may be combined with an administration of a Freund's complete adjuvant or a Freund's incomplete adjuvant for the purpose of enhancing the antibody-producing ability. The administration is performed usually once per 2 to 6 weeks, 2 to 10 times in total. A warm-blooded animal employed may for example be monkey, rabbit, dog, guinea-pig, mouse, rat, sheep, goat and chicken, with mouse and rat being employed preferably.

For preparing a monoclonal antibody-producing cell, antigen-immunized warm-blooded animals, for example mice, are screened for an individual exhibiting an antibody titre, from which a spleen or a lymph node is extracted 2 to 5 days after the final immunization, and an antibody-producing cell contained therein is fused with a homogeneous or heterogeneous myeloma cell, whereby preparing a monoclonal antibody-producing hybridoma. The antibody titre of an antiserum can be determined for example by reacting a labeled protein described below with the antiserum followed by determining the activity of the label bound to an antibody. The fusion can be accomplished by a known method such as one by KEHLER and MILLSTEIN (Nature, 256, 495 (1975)). A fusion promoting agent may for example be a polyethylene glycol (PEG) or Sendai virus, with PEG being employed preferably.

A myeloma cell may for example be a myeloma cell of a warm-blooded animal such as NS-1, P3U1, SP2/0 and AP-1, with P3U1 being employed preferably. A preferred ratio of the antibody-producing cell count (spleen cell count) and the myeloma cell count employed is about 1:1 to 20:1, and an efficient cell fusion is accomplished by adding a PEG (preferably PEG 1000 to PEG 6000) at a concentration of 10 to 80 % and incubating at 20 to 40°C, preferably 30 to 37°C, for 1 to 10 minutes.
While various methods are applicable in screening for a monoclonal antibody-producing hybridoma, those which may be exemplified are a method involving an addition of a hybridoma culture supernatant to a solid phase (e.g., a microplate) on which a protein antigen is adsorbed directly or in combination with a carrier followed by an addition of an anti-immunoglobulin labeled with a radioactive substance or with an enzyme (anti-mouse immunoglobulin antibody is employed when the cell employed in the cell fusion is a mouse cell) or protein A whereby detecting a monoclonal antibody bound to the solid phase, or a method involving an addition of a hybridoma culture supernatant to a solid phase on which an anti-immunoglobulin antibody or protein A followed by an addition of a protein labeled with a radioactive substance or with an enzyme whereby detecting a monoclonal antibody bound to the solid phase.

A monoclonal antibody can be selected by a method known per se or its modification. A HAT (hypoxanthine, aminopterin, thymidine)-supplemented medium for an animal cell culture is employed usually. A medium for the selection and the breeding may be any medium capable of growing a hybridoma. For example, an RPMI 1640 medium supplemented with 1 to 20 %, preferably 10 to 20 % fetal bovine serum, a GIT medium (WAKO PURE CHEMICAL INDUSTRIES, LTD.) supplemented with 1 to 10 % fetal bovine serum and a serum-free medium for incubating a hybridoma (SFM-101, NISSUI SEIYAKU CO., LTD.) may be employed. The incubation temperature is usually 20 to 40°C, preferably about 37°C. The incubation time is usually 5 days to 3 weeks, preferably 1 week to 2 weeks. The incubation may be performed usually under an atmosphere of 5% CO₂ gas. The antibody titre of a hybridoma incubation supernatant can be determined similarly to an antibody titre of an antiserum described above.

### (b) Purification of monoclonal antibody

A monoclonal antibody can be separated and purified by a method known per se such as a method for separating and purifying an immunoglobulin [e.g., salting out, alcohol precipitation, isoelectric precipitation, electrophoresis, ion exchanger (e.g., DEAE) adsorption and desorption, ultracentrifugation, gel filtration, a specific purification for collecting an antibody exclusively using an antigen-binding solid phase or an active adsorbent such as protein A or protein G followed by dissociating the binding to obtain the antibody].

### [Polyclonal antibody preparation]

A polyclonal antibody of the invention can be prepared by a method known per se or its modification. For example, an immune antigen (protein antigen) itself or a complex thereof with a carrier protein is produced and used as described above in the section of the polyclonal antibody preparation to immunize a warm-blooded animal, from which a material containing an antibody against a protein of the invention is isolated and purified to obtain an antibody.

With regard to a complex of an immune antigen with a carrier protein employed for immunizing a warm-blooded animal, the type of the carrier protein and the mixing ratio of the carrier and a hapten may vary provided that the antibody can be produced efficiently in relation to the hapten crosslinked to the carrier for the immunization, and any substance can be crosslinked at any ratio, and, in a typical method, about 0.1 to 20, preferably about 1 to 5 of parts by weight of bovine serum albumin, bovine thyroglobulin or hemocyanin is coupled to 1 parts by weight of a hapten.

For coupling a hapten to a carrier, various condensing agent can be employed, such as gurtaraldehyde or carbodiimide, a maleimide activated ester, an active ester reagent having a thiol group or a dithiopyridyl group.

A condensation product may can be administered as it is or in combination with a carrier or diluent to a site of a warm-blooded animal where the antibody can be produced. The administration may be combined with an administration of a Freund's complete adjuvant or a Freund's incomplete adjuvant for the purpose of enhancing the antibody-producing ability. The administration is performed usually once per 2 to 6 weeks, 3 to 10 times in total.

A polyclonal antibody can be collected from a blood, ascites, preferably from a blood, of a warm-blooded animal immunized as described above.

The polyclonal antibody titre of an antiserum can be determined similarly to the measurement of the antibody titre of an antiserum described above. A polyclonal antibody can be isolated and purified in accordance with a method for isolating and purifying an immunoglobulin similar to a method for isolating and purifying a monoclonal antibody described above.

An antisense DNA having a base sequence complementary with or substantially complementary with a DNA encoding a protein or a partial peptide of the invention (hereinafter such DNA is referred as a DNA of the invention in discussing an antisense DNA) may be any antisense DNA which has a base sequence complementary with or substantially complementary with a DNA of the invention and which has an ability of inhibiting the expression of such DNA.

A base sequence complementary with or substantially complementary with a DNA of the invention may for example be a base sequence having about 92 % or more, preferably about 95 % or more, more preferably about 98 % or more of the homology with the entire base sequence or a partial base sequence of a base sequence complementary with a DNA of the invention (i.e., a complemental strand of a DNA of the invention). One preferred particularly is an antisense DNA having about 70 % or more, preferably about 80 % or more, more preferably about 90 % or more, most preferably about 95 % or more of the homology with the complemental strand of the base sequence in the moiety encoding the N-terminal of a protein of the invention among the entire base sequence of the complemental strand of the DNA of the invention. Such antisense DNA can be produced by a known DNA synthesizer and the like.

Uses of a protein or a partial peptide of the invention or its salt (hereinafter sometimes abbreviated as a protein of the invention), a DNA encoding a protein or a partial peptide of the invention (hereinafter sometimes abbreviated as a DNA of the invention), an antibody against a protein or a partial peptide of the invention or its salt (hereinafter sometimes abbreviated as an antibody of the invention) and an antisense DNA are described below.

### (1) Agent for treating or preventing various diseases involving proteins of the invention

A protein of the invention and a DNA of the invention can be used as a pharmaceutical such as an agent for treating or preventing any of phosphodiesterase-related various diseases.

For example, in a patient whose in vivo phosphodiesterase V is reduced or deleted, the function of a protein of the invention can fully or normally be exerted in this patient by (i) administering a DNA of the invention to this patient to effect an in vivo expression of the protein of the invention, by (ii) inserting a DNA of the invention to a cell to express the protein of the invention followed by implanting this cell into the patient, or by (iii) administering the protein of the invention to this patient.

When a DNA of the invention is used as an agent for a treatment or a prevention, such DNA can be administered, directly or after an insertion into a suitable vector such as a retrovirus vector, an adenovirus vector, an adenovirus-associated virus vector and the like, to a human or a warm-blooded animal by a standard method. A DNA of the invention can be administered as it is or in a formulation together with a physiolgically acceptable carrier such as an auxiliary agent for promoting an ingestion using a gene gun or a catheter such as a hydrogel catheter.

A protein of the invention may be given orally as an optionally sugar-coated tablet, capsule, elixir, microcapsule and the like, or parenterally as a formulation for injection such as an aseptic solution or suspension in water or pharmacological acceptable liquid. For example, such formulation can be produced by mixing a protein of the invention with a physiologically acceptable carrier, flavor, excipient, vehicle, preservative stabilizer, binder and the like in a unit dosage form which is acceptable generally in a pharmaceutical practice. The amount of an active ingredient in such formulation should be adjusted to achieve a suitable dose within a specified range.

An additive which may be incorporated into a tablet or a capsule may for example be a binder such as gelatin, corn starch, tragacanth, gum arabic and the like, an excipient such as crystalline cellulose, an expander such as corn starch, gelatin, alginic acid and the like, a lubricant such as magnesium stearate, a sweetener such as sucrose, lactose or saccharin, a flavor such as peppermint, oil of Geultheria ovatifolia spp., cherry and the like. When a unit dosage form is a capsule, a liquid carrier such as a fat may further be incorporated in addition to the materials described above. An aseptic formulation for injection can be prepared in accordance with an ordinary pharmaceutical practice such as a dissolution or a suspension of an active ingredient, a naturally-occurring vegetable oil such as sesame oil and palm oil in a vehicle such as a water for injection.

A water for injection may for example be physiological saline, an isotonic solution containing glucose or other auxiliary agents (for example, D-sorbitol, D-mannitol, sodium chloride) and the like, which may be used in combination with an suitable solubilizer such as an alcohol (for example, ethanol), a polyalcohol (for example, propylene glycol, polyethylene glycol), a non-ionic surfactant (for example, polysorbate 80™, HCO-50). An oily liquid may for example be sesame oil and soybean oil, which may be used in combination with an solubilizer such as benzyl benzoate, benzyl alcohol and the like. In addition, a buffer agent (for example, phosphate buffer, sodium acetate buffer), a pain-ameliorating agent (for example, benzalkonium chloride, procaine hydrochloride), a stabilizer (for example, human serum albumin, polyethylene glycol), a preservative (for example, benzyl alcohol, phenol), an antioxidant may also be incorporated. A formulation for injection thus prepared is then filled usually in a suitable ampoule.

A vector into which a DNA of the invention is inserted may also be formulated as described above, and used usually via a parenteral route.

Since a formulation thus obtained is safe and less toxic, it can be administered to a human or a warm-blooded animal (for example, rat, mouse, guinea-pig, rabbit, bird, sheep, swine, cattle, horse, cat, dog, monkey and the like).

While a protein of the invention may be given at a dose which varies depending on the target disease, the subject and the administration route, it can be given orally to an adult (60 kg) usually at a daily dose as the protein of about 0.1 mg to 100 mg, preferably about 1.0 to 50 mg, more preferably about 1.0 to 20 mg. When it is given parenterally, the single dose of the protein may vary depending on the subject and the target disease, and may for example be given as a formulation for injection to an adult (60 kg) usually at a daily dose as the protein of about 0.1 mg to 30 mg, preferably about 0.1 to 20 mg, more preferably about 0.1 to 10 mg, which is injected to the site of a lesion. Also in other animals, a dose calculated for a 60 kg body weight may be administered.

### (2) Screening for pharmaceutical candidate against diseases

A compound inhibiting the functions of a protein of the invention (e.g., a phosphodiesterase activity, more preferably a phosphodiesterase V activity, most preferably, a phosphodiesterase activity specific to a protein having the amino acid sequence represented by SEQ. ID. NO:7, cGMP activity and the like) or its salt can for example be used as an agent for treating or preventing a circulatory disease (angina of effort or resting (coronary contractile angina or hetero-angina), hypertension, congestive heart insufficiency, pulmonary hypertension, atherosclerosis, reduced vascular patency. stenosis, peripheral vascular disease, cerebral apoplexy and the like), occlusive pulmonary disease (chronic asthma, bronchitis and the like), allergic disease (allergic asthma, allergic rhinitis, hives and the like), a disease characterized by the motility of a digestive tract (irritable bowel syndrome and the like), glomerular disease, tubular interstitial disease, renal failure, diabetic complication, glaucoma, erectile dysfunction in male mammalian animals including human, female dysfunction in female mammalian animals including human, premature birth, dysmenorrhea and the like.

Accordingly, a protein of the invention is useful as a reagent for screening for a compound inhibiting the functions of the protein of the invention or its salt.

Thus, the present invention provides:
(1) a method for screening for a compound promoting the functions of a protein or its partial peptide of the invention or its salt (e.g., a phosphodiesterase activity, more preferably a phosphodiesterase V activity, most preferably, a phosphodiesterase activity specific to a protein having the amino acid sequence represented by SEQ. ID. NO:7, cGMP activity and the like) or its salt (hereinafter sometimes abbreviated as a promoting agent) or a compound suppressing the functions of a protein or its partial peptide of the invention or its salt (e.g., a phosphodiesterase activity, more preferably a phosphodiesterase V activity, most preferably, a phosphodiesterase activity specific to a protein having the amino acid sequence represented by SEQ. ID. NO:7, cGMP activity and the like) or its salt (hereinafter sometimes abbreviated as a inhibiting agent) comprising a use of said protein, or its partial peptide of the invention or its salt, and more particularly, provides:
(2) a method for screening for a promoting agent or a inhibiting agent comprising a comparison between (i) a contact of a protein or its partial peptide of the invention or its salt with a substrate, and (ii) a contact of a protein or its partial peptide of the invention or its salt with a substrate and a test compound.

Typically in a screening method described above, the functions of a protein of the invention (for example, a phosphodiesterase activity, more preferably a phosphodiesterase V activity, most preferably, a phosphodiesterase activity specific to a protein having the amino acid sequence represented by SEQ. ID. NO:7, cGMP activity and the like) observed upon (i) and (ii) are determined and then compared.

As a substrate, any substance capable of serving as a substrate for a protein of the invention may be employed.

As a test compound, a peptide, a protein, a non-peptide compound, a synthetic compound, a fermentation product, a cell extract, a plant extract and an animal extract, which may be novel or known per se, may be exemplified, with cGMP being used preferably.

For practicing a screening method described above, a protein of the invention is suspended in a buffer suitable for the screening to prepare a standard of the protein of the invention. Such buffer may be any one which does not inhibit the reaction of a protein of the invention with a substrate, such as a phosphate buffer, a tris-HCl buffer at a pH of about 4 to 10 (preferably about 6 to 8).

The reaction temperature is 10 to 50°C (preferably 25°C to 37°C). The concentration of a protein of the invention is 0.1 µg/ml to 50 µg/ml (preferably 1 µg/ml to 25 µg/ml).

The activity of a protein of the invention (for example, a phosphodiesterase activity, more preferably a phosphodiesterase V activity, most preferably, a phosphodiesterase activity specific to a protein having the amino acid sequence represented by SEQ. ID. NO.7, cGMP activity and the like) can be determined by a known method (Thompson, W.J. et al., Biochemistry 10, 311 (1971), J.D. Johnson et al., Anal. Chem. 162, 291 (1987), R.J. Schilling et al., Anal. Chem. 216, 154 (1994), Phosphodiesterase [³H] cGM SPA enzyme assay kit (Amersham) and the like).
For example, a compound which gives an increase in the phosphodiesterase activity upon (ii) when compared with that upon (i) by about 20 % or more, preferably 30 % or more, more preferably about 50 % or more can be selected as a compound which promotes the activity of a protein of the invention, while a compound which gives a reduction in the phosphodiesterase activity upon (ii) when compared with that upon (i) by about 20 % or more, preferably 30 % or more, more preferably about 50 % or more can be selected as a compound which inhibits the activity of a protein of the invention.

A screening kit of the invention comprises a protein or its partial peptide of the invention or its salt. An example of a screening kit of the invention is described below.

### (Reagents for screening]

1. Assay buffer
   Tris-HCL buffer, pH 7.5 (containing MgCl₂, EGTA)
2. Standard protein
   Protein or its partial peptide of the invention or its salt
3. Substrate
   [³H]3',5'-cyclic guanosine monophosphate
4. Detection
   Radioactivity of hydrolyzed [³H]5'- guanosine monophosphate

### [Assay]

To a protein of the invention, [³H]3',5'-cyclic guanosine monophosphate is added and reacted at 30°C for 30 minutes and then the radioactivity of hydrolyzed [³H]5'- guanosine monophosphate, whereby determining a phosphodiesterase activity. Typically, Phosphodiesterase [³H] cGM SPA enzyme assay kit (Amersham) can be employed.
A compound obtained by a screening method or using a screening kit of the invention or its salt is a compound selected from the test compounds described above, for example, a peptide, a protein, a non-peptide compound, a synthetic compound, a fermentation product, a plant extract, an animal extract, plasma and the like, and is a compound which promotes or inhibits a function of the protein of the invention.

A salt of such compound is similar to a salt of a protein of the invention described above.

A compound inhibiting the functions of a protein of the invention (e.g., a phosphodiesterase activity, more preferably a phosphodiesterase V activity, most preferably, a phosphodiesterase activity specific to a protein having the amino acid sequence represented by SEQ. ID. NO:7, cGMP activity and the like) or its salt can for example be used as an agent for treating or preventing a circulatory disease (angina of effort or resting (coronary contractile angina or hetero-angina), hypertension, congestive heart insufficiency, pulmonary hypertension, atherosclerosis, reduced vascular patency, stenosis, peripheral vascular disease, cerebral apoplexy and the like), occlusive pulmonary disease (chronic asthma, bronchitis and the like), allergic disease (allergic asthma, allergic rhinitis, hives and the like), a disease characterized by the motility of a digestive tract (irritable bowel syndrome and the like), glomerular disease, tubular interstitial disease, renal failure, diabetic complication, glaucoma, erectile dysfunction in male mammalian animals including human, female dysfunction in female mammalian animals including human, premature birth, dysmenorrhea and the like.

A compound obtained by a screening method or using a screening kit of the invention can be employed as an agent for treating or preventing of the diseases described below in a customary manner. For example, similarly to a pharmaceutical containing a protein of the invention described above, a tablet, a capsule, an elixir, a microcapsule, an aseptic solution and a suspension may be formulated.

Since a formulation thus obtained is safe and less toxic, it can be administered to a human or a warm-blooded animal (for example, mouse, rat, rabbit, sheep, swine, cattle, horse, bird, cat, dog, monkey and the like).

While a compound or its salt may be given at a dose which varies depending on its effect, the target disease, the subject and the administration route, it can be given, in the case where a compound inhibiting a protein of the invention is administered orally for the purpose of treating a circulatory disease, to an adult (60 kg) usually at a daily dose as the compound of about 0.1 mg to 100 mg, preferably about 1.0 to 50 mg, more preferably about 1.0 to 20 mg. When it is given parenterally, the single dose of the compound may vary depending on the subject and the target disease, and may for example be given conveniently, in the case where a compound inhibiting a protein of the invention is administered orally for the purpose of treating a circulatory disease, as a formulation for injection to an adult (60 kg) usually at a daily dose as the protein of about 0.1 mg to 100 mg, preferably about 0.1 to 20 mg, more preferably about 0.1 to 10 mg, which is injected intravenously. Also in other animals, a dose calculated for a 60 kg body weight may be administered.

### (3) Quantification of protein or its partial peptide of the invention or its salt

Since an antibody against a protein of the invention (hereinafter sometimes abbreviated as an antibody of the invention) can recognize the protein of the invention specifically, it can be used for quantifying the protein of the invention in a test sample, especially by a sandwich immunoassay.

Thus, the present invention also provides:
(i) a method for quantifying a protein of the invention in a test sample comprising a competitive reaction of an antibody of the invention with the test sample and a labeled protein of the invention followed by a determination of the ratio of the labeled protein of the invention bound to the antibody; and,
(ii) a method for quantifying a protein of the invention in a test sample comprising a simultaneous or sequential reaction of the test sample with an antibody of the invention insolubilized on a carrier and a labeled other antibody of the invention followed by a determination of the activity of the label on the insolubilized carrier.

In a quantification method of (ii) described above, it is preferred that one antibody is an antibody recognizing the N-terminal of a protein of the invention and the other is reactive with the C-terminal of the protein of the invention.

Using a monoclonal antibody against a protein of the invention (hereinafter sometimes referred to as a monoclonal antibody of the invention), the protein of the invention can be quantified and a detection by a tissue staining and the like can also be performed. For these purposes, an antibody molecule itself may be used, or a F(ab')₂, Fab' or Fab fraction of the antibody molecule may also be employed.

A method for quantifying a protein of the invention using an antibody of the invention is not particularly limited, and may be any method in which the amount of an antibody, antigen or antibody-antigen complex corresponding to the amount of the antigen (for example, the amount of the protein) in a test sample is detected physically or chemically and then a calculation is made based on a standard curve obtained by using the standard solutions containing known amounts of the antigen. For example, a nephrometry, a competitive assay, an immunometric assay and a sandwich assay can preferably be employed, with a sandwich assay described below being preferred in view of the sensitivity and the specificity.

A label employed in an assay using a labeled substance may for example be a radioisotope, an enzyme, a fluorescent substance, a luminescent substance and the like. Such radioisotope may for example be [¹²⁵I], [¹³¹I] [³H], [¹⁴C] and the like. An enzyme described above is preferably one which is stable and has a high specific activity and may for example be β-galactosidase, β- glucosidase, alkaline phosphatase, peroxidase, malate dehydrogenase and the like. A fluorescent substance may for example be FLUORESCAMINE, fluorescein isothiocyanate and the like. A luminescent susbtance may for example be luminol, a luminol derivative, luciferin, lucigenin and the like. A biotin-avidin system may also be employed for binding a label to an antibody or an antigen.

An insolubilization of an antigen or an antibody may be effected utilizing a physical adsorption, and a chemical binding employed usually for insolubilizing a protein or an enzyme may also be employed. A carrier may for example be an insoluble polysaccharide such as agarose, dextran and cellulose, a synthetic resin such as polystyrene, polyacrylamide and silicon, as well as a glass.

In a sandwich method, an insolubilized inventive monoclonal antibody is reacted with a test sample (primary reaction), and then a labeled other monoclonal antibody of the invention is reacted (secondary reaction), and then the activity of the label on the insolubilized carrier is determined, whereby quantifying a protein of the invention in the test sample. The primary reaction and the secondary reaction can be performed in the opposite order or may be performed simultaneously or at an interval. A label and a method for the insolubilization are as described above. It is not necessary always in a sandwich immunoassay that an antibody employed as an antibody for a solid phase or an antibody for labeling is of a single type, and several antibodies can be employed as in a mixture for the purpose of a higher sensitivity of the measurement.

In a method for determining a protein of the invention by a sandwich method of the invention, monoclonal antibodies employed in the primary reaction and the secondary reaction are preferably those differing from each other in the site of the binding to the protein of the invention. Thus, the antibody employed in the primary and secondary reactions are selected so that when the antibody employed in the secondary reaction recognizes the C-terminal of the protein of the invention then the antibody employed in the primary reaction recognizes the sites other than the C-terminal, such as the N-terminal.

A monoclonal antibody can be used in an assay system other than a sandwich assay, such as a competitive assay, an immunometric assay and a nephrometry.

In a competitive assay, an antigen in a test sample and a labeled antigen are reacted competitively with an antibody and then unreacted labeled antigen (F) is separated from an antibody-binding labeled antigen (B) (B/F separation), and the amount of the label on either B or F is determined, whereby quantifying the antigen in the test sample. This reaction is conducted by a liquid phase method employing a soluble antibody as an antibody and performing a B/F separation using a polyethylene glycol and a secondary antibody, and also by a solid phase method employing a solid phase antibody as a primary antibody or employing a soluble antibody as a primary antibody and a solid phase antibody as a secondary antibody.

In an immunometric method, an antigen in a test sample and a solid phase antigen are reacted competitively with a certain amount of a labeled antibody, and then the solid phase is separated from a liquid phase, or an antigen in a test sample is reacted with a labeled antibody in excess and then a solid phase antigen is added to bind an unreacted labeled antibody to the solid phase and subsequently the solid phase is separated from a liquid phase. Then the amount of the label in either phase is determined, whereby quantifying the antigen in the test sample.

In an nephrometric assay, the amount of an insoluble precipitate formed as a result of an antigen-antibody reaction in a gel or a solution is determined. Even when the amount of an antigen in a test sample is very small and only a small amount of a precipitation can be obtained, a laser nephrometry utilizing a scattering of the laser is employed preferably.

When applying each immunological assay described above to a quantification method of the invention, no particular condition or operation is specified. Ordinary condition and operation in each method may be employed in combination with a technology known by those skilled in the art to construct an assay system for a protein of the invention. Such general technology is found in corresponding textbooks or guidebooks.

For example, "Radioimmunoassay", ed. by H.Irie (KODANSHA, 1974), "Radioimmunoassay II", ed. by H.Irie (KODANSHA, 1979), "Enzyme Immunoassay", ed. by E.Ishikawa et al., (IGAKUSHOIN, 1978), "Enzyme Immunoassay", (2nd Volume), ed. by E.Ishikawa et al., (IGAKUSHOIN, 1982), "Enzyme Immunoassay", (3rd Volume), ed. by E.Ishikawa et al., (IGAKUSHOIN, 1987), "Methods in Enzymology", Vol.70, (Immunochemical Techniques (Part A)), Vol.73, (Immunochemical Techniques (Part B)). Vol.74, (Immunochemical Techniques (Part C)), Vol.84, (Immunochemical Techniques (Part D: Selected Immunoassays)), Vol.92, (Immunochemical Techniques (Part E: Monoclonal Antibodies and General Immunoassay Methods)), Vol.121, (Immunochemical Techniques (Part I: Hybridoma Technology and Monoclonal Antibodies)). (Academic Press).

As described above, a protein of the invention can be quantified at a high sensitivity by employing an antibody of the invention.

Furthermore, when a determination of the concentration of a protein of the invention using an antibody of the invention revealed an increase in the protein of the invention, then there is a diagnosis of a high risk of a current or future occurrence of a disease such as a circulatory disease (angina of effort or resting (coronary contractile angina or hetero-angina), hypertension, congestive heart insufficiency, pulmonary hypertension, atherosclerosis, reduced vascular patency, stenosis, peripheral vascular disease, cerebral apoplexy and the like), occlusive pulmonary disease (chronic asthma, bronchitis and the like), allergic disease (allergic asthma, allergic rhinitis, hives and the like), a disease characterized by the motility of a digestive tract (irritable bowel syndrome and the like), glomerular disease, tubular interstitial disease, renal failure, diabetic complication, glaucoma, erectile dysfunction in male mammalian animals including human, female dysfunction in female mammalian animals including human, premature birth, dysmenorrhea and the like.

An antibody of the invention can also be employed for detecting a protein of the invention which is present in a test sample such as a body fluid or a tissue. It can also be employed in preparing an antibody column used for purifying a protein of the invention, in detecting a protein of the invention in each fraction upon a purification, or in analyzing the behavior of a protein of the invention in a test cell.

### (4) Gene diagnostic agent

Since a DNA of the invention, when used, for example, as a probe, can detect an abnormality (gene abnormality) in a DNA or a mRNA encoding a protein or its partial peptide of the invention in a human or a warm-blooded animal (for example, rat, mouse, guinea-pigs, rabbit, bird, sheep, swine, cattle, horse, cat, dog, monkey and the like), it is useful in a gene diagnosis of an impairment, a mutation or a reduced expression of such DNA or mRNA as well as an increase or an increased expression of such DNA or mRNA.

A gene diagnosis employing a DNA of the invention can be performed for example by a northern hybridization known per se or a PCR-SSCP method (Genomics, Vol.5, p874-879 (1989)) or a method described in Proceedings of the National Academy of Sciences of USA, Vol.86, p2766-2770 (1989).

For example, an increased expression of such mRNA detected in a northern hybridization indicates a high risk of a disease such as a circulatory disease (angina of effort or resting (coronary contractile angina or hetero-angina), hypertension, congestive heart insufficiency, pulmonary hypertension, atherosclerosis, reduced vascular patency, stenosis, peripheral vascular disease, cerebral apoplexy and the like), occlusive pulmonary disease (chronic asthma, bronchitis and the like), allergic disease (allergic asthma, allergic rhinitis, hives and the like), a disease characterized by the motility of a digestive tract (irritable bowel syndrome and the like), glomerular disease, tubular interstitial disease, renal failure, diabetic complication, glaucoma, erectile dysfunction in male mammalian animals including human, female dysfunction in female mammalian animals including human, premature birth, dysmenorrhea and the like.

### (5) Antisense DNA-containing pharmaceutical

Since an antisense DNA which can be bound complementarily to a DNA of the invention and which can suppresses the expression of such DNA can suppress an in vivo function of a protein of the invention or a DNA of the invention, it can be used as an agent for treating or preventing a disease such as a circulatory disease (angina of effort or resting (coronary contractile angina or hetero-angina), hypertension, congestive heart insufficiency, pulmonary hypertension, atherosclerosis, reduced vascular patency, stenosis, peripheral vascular disease, cerebral apoplexy and the like), occlusive pulmonary disease (chronic asthma, bronchitis and the like), allergic disease (allergic asthma, allergic rhinitis, hives and the like), a disease characterized by the motility of a digestive tract (irritable bowel syndrome and the like), glomerular disease, tubular interstitial disease, renal failure, diabetic complication, glaucoma, erectile dysfunction in male mammalian animals including human, female dysfunction in female mammalian animals including human, premature birth, dysmenorrhea and the like.

When an antisense DNA described above is used as an agent for treating or preventing a disease described above, it can be used similarly to an inventive DNA-containing agent mentioned above for treating or preventing a disease.

For example, when such antisense DNA is employed, it can be subjected to a standard method as it is or after an insertion to a suitable vector such as a retrovirus vector, an adenovirus vector, an adenovirus-associated virus vector and the like. The antisense DNA can be administered as it is or in a formulation together with a physiolgically acceptable carrier such as an auxiliary agent for promoting an ingestion using a gene gun or a catheter such as a hydrogel catheter.

In addition, such antisense DNA can be used also as a diagnostic oligonucleotide probe for investigating the presence or the expression state of a DNA of the invention in a tissue or a cell.

### (6) DNA transfer animal

The present invention provides a DNA encoding an exogenous protein of the invention (hereinafter referred to as of the invention), a recombinant vector.

A non-human mammalian animal having an exogenous DNA of the invention or its variant (hereinafter referred to as a DNA transfer animal of the invention) can be produced by transferring an intended DNA to an embryonic cell including an unfertilized ovum, a fertilized ovum, a sperm or a primordial cell thereof, preferably an developing embryo of a non-human mammalian animal (more preferably in a unicellular or fertilized ovular phase generally not later than 8-cell phase) using a calcium phosphate method, an electric pulse method, a lipofection method, an aggregation method, a microinjection method, a particle gun method, a DEAE-dextran method and the like. Also by such DNA transfer method, an intended exogenous DNA of the invention is transferred to a somatic cell and an in vivo organ or tissue cell which is then utilized in a cell culture or a tissue culture or which may be fused with an embryonic cell described above by a cell fusion method known per se, whereby obtaining an inventive DNA transfer animal.

A non-human mammalian animal may for example be a cattle, swine, sheep, goat, rabbit, dog, cat, guinea-pig, hamster, mouse, rat and the like. Among these animals, a rodent animal, especially a mouse (for example, a pure line such as C57BL/6 line and DBA2 line, a hybrid line such as B6C3F1 line, BDF1 line, B6D2F1 line, BALB/c line and ICR lines), whose individual development and biological cycle are relatively short-spanned and which can readily be fertilized, is preferred in view of the preparation of an animal model system.

A "mammalian animal" referred here in conjunction with a recombinant vector capable of being expressed in a mammalian animal may for example be a human in addition to non-human animals described above.

An exogenous DNA of the invention means a DNA of the invention once isolated or extracted from a mammalian animal rather than a DNA of the invention which is possessed originally by a non-human mammalian animal.

A DNA variant of the invention is a DNA resulting from a variation (for example, mutation) occurring in the base sequence of an original DNA of the invention, typically, a DNA undergoing an addition, deletion or substitution of a base as well as an abnormal DNA.

Such abnormal DNA means a DNA which expresses an abnormal protein of the invention, for example, a DNA which expresses a protein inhibiting a function of a normal protein of the invention.

An exogenous DNA of the invention may be derived from a mammalian animal which is homogeneous or heterogeneous to a target animal. For transferring a DNA of the invention to a target animal, a use as a DNA construct bound downstream of a promoter capable of expressing such DNA is advantageous generally. For example, a DNA of the invention can be transferred by microinjecting a DNA construct (e.g., vector) bound to a human DNA of the invention to a fertilized ovum of a target mammalian animal, such as a mouse fertilized ovum, downstream of any promoter capable of expressing a DNA derived from any mammalian animal (for example, rabbit, dog, cat, guinea-pig, hamster, rat, mouse and the like) having a DNA of the invention whose homology with human DNA of the invention is high, whereby obtaining a DNA transfer mammalian animal which highly expresses the DNA of the invention.

An expression vector of a protein of the invention employed here is an E.coli-derived plasmid, a B.subtilis-derived plasmid, an yeast-derived plasmid, a bacteriophage such as λ phage, a retrovirus such as Moloney's leukemia virus, an animal virus such as vaccinia virus or baculovirus and the like. Among those listed above, an E.coli-derived plasmid, a B.subtilis-derived plasmid and an yeast-derived plasmid are preferred.

A promoter for regulating the expression of a DNA described above may for example be [1] a promoter of a DNA derived from a virus (e.g., simian virus, cytomegalovirus, Moloney's leukemia virus, JC virus, mammary cancer virus, polio virus and the like), [2] a promoter derived from any of various mammalian animals (human, rabbit, dog, cat, guinea-pig, hamster, rat, mouse and the like), such as a promoter of albumin, insulin II, UROPLAKIN II, erastase, erythropoietin, endoserine, myocreatine kinase, glia fibrous acidic protein, glutathione S-transferase, platelet-derived growth factor β, keratin K1, K10 and K14, collagen type I and II, cyclic AMP-dependent protein kinase βI subunit, dystrophin, tartarate-resistant alkaline phosphatase, atrial sodium diuretic factor, endothelial receptor tyrosine kinase (generally designated as Tie2), sodium potassium adenosin triphosphorylase (Na,K-ATPase), neurofilament light chain, metallothionein I and IIA, metalloproteinase 1 tissue inhibitor, MHC class I antigen (H-2L), Hras, renin, dopamine β-hydroxylase, thiroid peroxidase (TPO), polypeptide chain elongation factor 1α (EF-1α), β-actin, α and β myosin heavy chain, myosin light chain 1 and 2, myelin basal protein, thyroglobulin, Thy-1, immunoglobulin, H chain variable part (VNP), serum amyloid P component, myoglobin, troponin C, smooth muscle α actin, preproencephalin A, basopressin and the like. Among those listed above, a systemically highly expressible cytomegalovirus promoter, a promoter of human polypeptide chain elongation factor 1α (EF-1α), human and avian β-actin promoter are employed preferably.

A vector described above preferably has a sequence which terminates the transcription (referred generally as terminator) of an intended mRNA in a DNA transfer mammalian animal, and may for example be a sequence of each DNA derived from viruses and mammalian animals, with SV40 terminator of a simian virus being employed preferably.

Otherwise, for the purpose of achieving a further higher expression of an intended exogenous DNA, a part of a splicing signal of each DNA, an enhancer region and a part of an eukaryotic DNA intron may be ligated 5' upstream of a promoter, between a promoter region and a translation region or 3' downstream of a translation region as appropriate.

The translation region of a normal protein of the invention can be obtained as an entire or part of a genome DNA from a DNA derived from liver, kidney, thyroid cell and fibroblast cell obtained from a human or any of mammalian animal (for example, rabbit, dog, cat, guinea pig, hamster, rat, mouse and the like) or from various commercial genome DNA libraries, or can also be obtained from a starting material which is a complementary DNA prepared by a known method from an RNA derived from liver, kidney, thyroid cell and fibroblast. An abnormal exogenous DNA serves to prepare a translation region involving a variation caused by subjecting a translation region of a normal protein obtained from a cell or a tissue described above to a point mutation-inducing procedure.

Such translation region can be prepared by an ordinary DNA engineering method ligating downstream of a promoter described above or, if necessary, upstream of a translation termination site as a DNA construct capable of being expressed in a transfer animal.

The transfer of an exogenous DNA of the invention in a phase of a fertilized ovum is ensured to be present all over the embryonic cell and the somatic cell of a target mammalian animal. The presence of an exogenous DNA of the invention in an embryonic cell of an engineered animal after a DNA transfer means that all of the posterior generations of the engineered animal have the exogenous DNA of the invention in all of their embryonic cells and somatic cells. A later generation of such animal which took over the exogenous DNA of the invention has the exogenous DNA in all of its embryonic cells and somatic cells.

A non-human mammalian animal to which an exogenous normal DNA of the invention has been transferred can, once after ensuring that the exogenous DNA is kept stably after a mating, be grown inheritably as an animal having such DNA in an ordinary breeding environment.

The transfer of an exogenous DNA of the invention in a phase of a fertilized ovum is ensured to be present in excess all over the embryonic cell and the somatic cell of a target mammalian animal. The presence of an exogenous DNA of the invention in an embryonic cell of an engineered animal after a DNA transfer means that all of the later generations of the engineered animal have the exogenous DNA of the invention in excess in all of their embryonic cells and somatic cells. A later generation of such animal which took over the exogenous DNA of the invention has the exogenous DNA in excess in all of its embryonic cells and somatic cells.

By obtaining a homozygote animal having a transduced DNA in both of the homologous chromosomes followed by mating both sexes of this animal, an inheritable breeding to allow all later generations to have such DNA in excess is possible.

A non-human mammalian cell having a normal DNA of the invention highly expresses the normal DNA of the invention, and a promotion of an intrinsic normal DNA may lead finally to a hyperactivity of the protein of the invention, whereby providing a corresponding pathological model animal. For example, an inventive normal DNA transfer animal is employed to investigate the mechanisms of a hyperactivity of a protein of the invention and a disease involving a protein of the invention and also to search for a method for treating such disease.

Since a mammalian animal to which an inventive exogenous normal DNA has been transferred exhibits an increase in a free form of a protein of the invention, it can also be utilized in screening for a therapeutic agent against a disease related to the protein of the invention.

On the other hand, a non-human mammalian animal having an exogenous abnormal DNA of the invention can, once after ensuring that the exogenous DNA is kept stably after a mating, be grown inheritably as an animal having such DNA in an ordinary breeding environment. In addition, an intended exogenous DNA can be used as a starting material after being integrated into a plasmid described above. A DNA construct with a promoter can be prepared by an ordinary DNA engineering method. The transfer of an abnormal DNA of the invention in a phase of a fertilized ovum is ensured to be present all over the embryonic cell and the somatic cell of a target mammalian animal. The presence of an abnormal DNA of the invention in an embryonic cell of an engineered animal after a DNA transfer means that all of the later generations of the engineered animal have the abnormal DNA of the invention in all of their embryonic cells and somatic cells. A later generation of such animal which took over the exogenous DNA of the invention has the abnormal DNA in all of its embryonic cells and somatic cells. By obtaining a homozygote animal having a transduced DNA in both of the homologous chromosomes followed by mating both sexes of this animal, an inheritable breeding to allow all later generations to have such DNA is possible.

A non-human mammalian cell having an abnormal DNA of the invention highly expresses the abnormal DNA of the invention, and a promotion of an intrinsic normal DNA may lead finally to a refractoriness due to the dysfunction of the protein of the invention, whereby providing a corresponding pathological model animal. For example, an inventive abnormal DNA transfer animal is employed to investigate the mechanisms of a refractoriness due to the dysfunction of the protein of the invention and also to search for a method for treating such disease.

In a typical use, an animal exhibiting a high expression of an abnormal DNA of the invention serves as a model for investigating an inhibitory effect of an abnormal protein of the invention on the function of a normal protein (dominant negative effect) in a case of a refractoriness due to the dysfunction of the protein of the invention.

Since a mammalian animal to which an inventive exogenous abnormal DNA has been transferred exhibits an increase in a free form of a protein of the invention, it can also be utilized in screening for a therapeutic agent against a refractoriness due to the dysfunction of the protein of the invention.

Other possible uses of the two DNA transfer animals of the invention may for example be:
[1] a use as a cell source for a tissue culture;
[2] an analysis of the relationship with a protein expressed or activated specifically by a protein of the invention by means of a direct analysis of a DNA or an RNA in a tissue of a DNA transfer animal of the invention or an analysis of a protein tissue expressed by a DNA;
[3] an investigation of a function of a cell derived from a tissue which is difficult generally to be cultured using a cell of a tissue having a DNA which is incubated by a standard tissue culture technology;
[4] a screening for an agent promoting a function of a cell using a cell described in Section [3]; and,
[5] a purification of a variant protein of the invention and a preparation of an antibody thereto.

In addition, it is possible by using a DNA transfer animal of the invention to investigate the clinical condition of a disease related to a protein of the invention such as a refractoriness due to the dysfunction of the protein of the invention and also possible to obtain further detailed pathological findings in each organ in a model of a disease relating to a protein of the invention, resulting in a contribution to a development of a new therapeutic method as well as an investigation and a treatment of a condition secondary to such disease.

It is also possible that each organ is extracted from a DNA transfer animal of the invention and then sliced and treated with a protease such as trypsin to obtain a free DNA transfer cell which may be incubated or classified into a culture cell line. A further use can be made in an identification of an inventive protein-producing cell, an investigation of the relationship with apoptosis, differentiation or proliferation as well as a signal transmission mechanism involved therein, and an investigation of an abnormality therein, thus providing a useful tool in studying a protein of the invention and its effects.

In addition, a DNA transfer animal of the invention can also be employed to provide a method for screening for an effective and readily-acting therapeutic agent against a disease related with a protein of the invention including a refractoriness due to the dysfunction of a protein of the invention using a test or an assay described above for the purpose of developing such therapeutic agent. Furthermore, a DNA therapy against a disease related with a protein of the invention can be investigated and developed by using a DNA transfer animal or an exogenous DNA expression vector of the invention.

### (8) Knock out animal

The present invention also provides an embryonic stem cell of a non-human mammalian animal in which a DNA of the invention is inactivated and a non-human mammalian animal whose expression of a DNA of the invention is insufficient.

Thus, the present invention provides:
(1) a non-human mammalian embryonic cell in which a DNA of the invention is inactivated;
(2) an embryonic stem cell according to Section (1) wherein said DNA is inactivated by transducing a reporter gene (e.g., an E.coli-derived β-galactosidase gene);
(3) an embryonic stem cell according to Section (1) which is neomycin resistant;
(4) an embryonic stem cell according to Section (1) wherein said non-human mammalian animal is a rodent animal;
(5) an embryonic stem cell according to Section (4) wherein said rodent animal is a mouse;
(6) a non-human mammalian animal in which a DNA of the invention is inactivated;
(7) a non-human mammalian animal according to Section (6) wherein said DNA is inactivated by transducing a reporter gene (e.g., an E.coli-derived β-galactosidase gene) and said reporter gene can be expressed under the regulation by a promoter for a DNA of the invention;
(8) a non-human mammalian animal according to Section (6) wherein said non-human mammalian animal is a rodent animal;
(9) a non-human mammalian animal according to Section (8) wherein said rodent animal is a mouse;
(10) a method for screening for a compound which promotes or inhibits a promoter activity for a DNA of the invention or its salt, comprising administering a test substance to an animal described in Section (7) and detecting an expression of a reporter gene.

A non-human mammalian embryonic stem cell in which a DNA of the invention is inactivated means a non-human mammalian embryonic stem cell (hereinafter abbreviated as ES cell) obtained by imparting a DNA of the invention possessed by said non-human mammalian animal with an artificial variation to suppress the DNA expression ability or by causing a substantial loss of the activity of a protein of the invention encoded by said DNA to yield a DNA having substantially no ability of expressing a protein of the invention (hereinafter sometimes referred to as a knockout DNA of the invention).

A non-human mammalian animal may be one of those described above.

A method for imparting a DNA of the invention with an artificial variation may for example be an gene engineering method for deleting a part or all of the DNA sequence or for inserting or substituting other DNA. As a result of such variation, the reading frame of a codon may be shifted or the function of a promoter or an exon is destructed, whereby producing a knockout DNA of the invention.

A non-human mammalian embryonic stem cell in which a DNA of the invention is inactivated (hereinafter abbreviated as a DNA-inactive ES cell of the invention or a knockout ES cell of the invention) may typically be obtained for example in a method in which a DNA of the invention possessed by an intended non-human mammalian animal is isolated, and into its exon moiety a drug resistant gene such as a neomycin resistant gene or a hygromycin resistant gene, or a reporter gene such as lacZ (β-galactosidase gene) and cat (chloramphenicol acetyl transferase gene) is inserted to destruct the function of the exon, or a DNA sequence terminating the transcription of the gene (for example, a polyA-carrying signal) is inserted into an intron moiety between the exons to disable a complete messenger RNA synthesis to yield a DNA chain having a DNA sequence constructed to lead finally to a gene destruction (hereinafter abbreviated as a targeting vector), which is then transduced into a chromosome of said animal for example by means of a homologous recombination to obtain an ES cell, which is screened for an inventive knockout ES cell by a southern hybridization employing a DNA sequence of the DNA of the invention or its proximity as a probe or by a PCR method using a DNA sequence on a targeting vector and a DNA sequence in a proximal region other than the DNA of the invention employed for preparing the targeting vector as primers.

An original ES cell in which aDNA of the invention is inactivated by a homologous recombination may for example be one which has already been established such as those described above or one which is newly established by a known method by Evans and Kaufma. While a 129 line ES cell is usually employed in these days as a mouse ES cell, it has no clear immunological background and thus may be replaced with one established using a BDF1 mouse (F1 of C57BL/6 and DBA/2) produced to achieve an improvement in the number of ovums recovered, which is too small in a C57BL/6 mouse or a C57BL/6 mouse, by mating with a DBA/2 mouse, for the purpose of obtaining an ES cell substitute which is of a pure line and whose genetic background is clarified immunologically. Since a BDF1 mouse has advantageous characteristics which are not only a large number of ovums being recovered and a durable ovum but also a background of a C57BL/6 mouse, an ES cell obtained using it can advantageously be employed to replace the genetic background of an engineered pathological model mouse with that of a C57BL/6 mouse by means of a back-crossing with the C57BL/6 mouse.

While a blastcyst on the 3.5the day after fertilization is employed generally for establishing an ES cell, otherwise an embryo in the 8-cell phase may also be recovered and incubated until a blastcyst, whereby obtaining a large number of the embryos in early phases.

ES cells of both sexes may be employed, and a male ES cell is advantageous generally for producing a chimera animal of a reproduction line. An earlier sexing is preferable for reducing the troublesome jobs in a complicated incubation.

Such sexing of an ES cell may be accomplished for example by performing a PCR method to amplify and detect a sex determinant region of a Y chromosome. By this procedure, a nucleus typing can be completed only with about one colony of the ES cells (about 50 cells) in contrast to a conventional procedure requiring about 10⁶ cells, thus allowing a primary selection of the ES cell at an early stage of the incubation to be conducted as such sexing step and enabling an earlier selection of male cells, resulting in a significant reduction in the troublesome jobs in the initial incubation.

As a secondary selection, an identification of the number of the chromosomes by a G-banding method may be performed. While the number of the chromosomes obtained is preferably 100 % of the normal number, the gene of an ES cell may first be knocked out and then cloned again to a normal cell (for example the number 2n of the chromosomes, for example in a mouse, is 40) if there is any difficulty in obtaining such number because of a physical reason associated with establishing the cell.

While a blastcyst thus obtained usually exhibits a satisfactory proliferation, it tends to lose an ability of an individual development and should be subjected to a careful subculture. For example, it is incubated on a suitable feeder cell such as an STO fibroblast in the presence of an LIF (1 to 1000 U/ml) in a CO₂ gas incubator (preferably in 5 % CO₂ gas and 95 % air, or 5 % CO₂ gas and 90 % air) at about 37°C, and, upon a subculture, it is treated for example with a trypsin/EDTA solution (generally 0.001 to 0.5 % trypsin/0.1-5 mM EDTA, preferably about 0.1 % trypsin/1mM EDTA) to divide into single cells, which are then inoculated onto a newly provided feeder cell. Such subculture is performed usually at an interval of 1 to 3 days, during which the cell is examined visually for any morphological abnormality, a cell having which is better to be discarded.

An ES cell is incubated as a monolayer until a high cell density is achieved under a suitable condition, or is incubated as a cell suspension until a cell aggregation is formed, whereby differentiating into various cell types such as parietal muscle, visceral muscle and cardiac muscle (M.J. Evans and M.H. Kaufman, Nature, Vol.292, p.154 (1981); G.R. Martin Proc. Natl. Acad. Sci. USA, Vol.78, p.7634 (1981); T.C.Doetschman et al., Journal of Embryology and Experimental Morphology, Vol.87, p.27, (1985)), and a DNA expression-insufficient cell of the invention obtained by differentiating an ES cell of the invention is useful in investigating an in vitro cellular biology of a protein of the invention.

A DNA expression-insufficient non-human mammalian animal of the invention can be distinguished from a normal animal by determining the amount of a mRNA in such animal by a known method and then comparing the amount indirectly.

Such non-human animal may be those listed above.

In a DNA expression-insufficient non-human mammalian animal of the invention, a DNA of the invention can be knocked out for example by transducing a targeting vector prepared as described above into a mouse embryonic stem cell or a mouse oval cell and then subjecting a DNA sequence formed by an inactivation of the DNA of the invention in the targeting vector as a result of such transduction to a homologous gene recombination with the DNA of the invention on a chromosome in the mouse embryonic stem cell or the mouse oval cell.

A cell in which a DNA of the invention is knocked out can be identified by a southern hybridization employing a DNA sequence of the DNA of the invention or its proximity as a probe or by a PCR method using a DNA sequence on a targeting vector and a DNA sequence in a proximal region other than the DNA of the invention derived from a mouse employed for preparing the targeting vector as primers. When a non-human mammalian animal embryonic stem cell is employed, a homologous gene recombination is employed to clone a cell line in which a DNA of the invention is inactivated, and the cell obtained is injected into a non-human mammalian animal embryo or blastcyst in a suitable phase, such as 8-cell phase, and a chimera embryo thus obtained is then implanted into an uterus of this non-human mammalian animal imparted with a pseudo-pregnancy. An animal thus produced is a chimera animal consisting of the both of a cell having a normal inventive DNA locus and a cell having an artificially varied inventive DNA locus.

When a part of the reproductive cells of the chimera animal described above has a varied inventive DNA locus, a group of individual animals obtained by mating this chimera with a normal animal is screened for an individual whose all tissues consist of the cells having the artificially varied inventive DNA loci, for example, by an assessment of a coat color. The individual thus obtained is usually an inventive protein heterexpression-impaired individual, which is mated with each other to obtain a newborn, from which an inventive protein homoexpression-impaired individual can be obtained.
When an oval cell is employed, a transgenic non-human mammalian animal into whose chromosome a targeting vector is transduced by injecting a DNA solution into the nucleus of the oval cell by a microinjection method can be obtained, and by comparing with such transgenic non-human mammalian animal and employing a homologous gene recombination an individual having a variation in a DNA locus of the invention is selected.

An individual in which a DNA of the invention is knocked out can, once after ensuring that the DNA is knocked out in an individual obtained by mating, be grown inheritably in an ordinary breeding environment.

A reproductive line may be obtained and maintained also by a standard method. Thus, by mating the both sexes of an animal having such inactivated DNA, a homozygote animal having such inactivated DNAs in both of the homologous chromosomes can be obtained. A homozygote animal obtained can be produced efficiently by growing in a condition employing one normal individual and several homozygotes per maternal animal. By mating the both sexes of a heterozygote animal, such inactivated DNA-possessing homozygote and heterozygote animals can be reproduced inheritably.

A non-human mammalian animal embryonic stem cell in which a DNA of the invention is inactivated is useful extremely in producing an inventive DNA expression-insufficient non-human mammalian animal.

Since an inventive DNA expression-insufficient non-human mammalian animal of the invention lacks various biological activities induced by a protein of the invention, it can serve as a model of a disease caused by an inactivation of the protein of the invention, thus being useful in an investigation of the causes of such disease or a development of a therapeutic method.

In the description and the drawings, a base or an amino acid may be designated as a code based on IUPAC-IUB, Commission on Biological Nomenclature or as a customary abbreviation in the art as exemplified below. When an amino acid is present as an optical isomer, it is in L form unless otherwise specified.
- DNA: : Deoxyribonucleic acid
- cDNA: : Complementary deoxyribonucleic acid
- A: : Adenine
- T: : Thymine
- G: : Guanine
- C: : Cytosine
- RNA: : Ribonucleic acid
- mRNA: : Messenger ribonucleic acid
- dATP: : Deoxyadenosine triphosphate
- dTTP: : Deoxythymidine triphosphate
- dGTP: : Deoxyguanosine triphosphate
- dCTP: : Deoxycytidine triphosphate
- ATP: : Adenosine triphosphate
- EDTA: : Ethylenediamine tetraacetic acid
- SDS: : Sodium dodecylphosphate
- Gly: : Glycine
- Ala: : Alanine
- Val: : Valine
- Leu: : Leucine
- Ile: : Isoleucine
- Ser: : Serine
- Thr: : Threonine
- Cys: : Cysteine
- Met: : Methionine
- Glu: : Glutamic acid
- Asp: : Aspartic acid
- Lys: : Lysine
- Arg: : Arginine
- His: : Histidine
- Phe: : Phenylalanine
- Tyr: : Tyrosine
- Trp: : Tryptophan
- Pro: : Proline
- Asn: : Asparagine
- Gln: : Glutamine
- pGlu: : Pyroglutaminic acid

Substituents, protective groups and reagents employed frequently in the specification are represented by the following codes.
- Me: : Methyl group
- Et: : Ethyl group
- Bu: : Butyl group
- Ph: : Phenyl group
- TC: : Thiazolidine-4(R)-carboxamide group
- Tos: : p-Toluenesulfonyi
- CHO: : Formyl
- Bzl: : Benzyl
- C₁₂Bzl: : 2,6-Dichlorobenzyl
- Bom: : Benzyloxymethyl
- Z: : Benzyloxycarbonyl
- Cl-Z: : 2-Chlorobenzyloxycarbonyl
- Br-Z: : 2-Bromobenzyloxycarbonyl
- Boc: : t-Butoxycarbonyl
- DNP: : Dinitrophenyl
- Trt: : Trityl
- Bum: : t-Butoxymethyl
- Fmoc: : N-9-Fluorenylmethoxycarbonyl
- HOBt: : 1-Hydroxybenztriazole
- HOOBt: : 3,4-Dihydro-3-hydroxy-4-oxo-1,2,3-benzotriazine
- HONB: : 1-Hydroxy-5-norbornene-2,3-dicarboxyimide
- DCC: : N,N'-Dicyclohexylcarbodiimide

The SEQ. ID. No. in the sequence listing in this specification represent the following sequences.
[SEQ. ID. No:1]
   An amino acid sequence substantially identical to the amino acid sequence of a human lung-derived phosphodiesterase V of the invention represented by SEQ. ID. NO:7
[SEQ. ID. No:2]
   An amino acid sequence resulted from the deletion of 109 amino acids starting from the N-terminal of the amino acid sequence represented by SEQ. ID. NO:1
[SEQ. ID. No:3]
   A base sequence of a DNA encoding the amino acid sequence represented by SEQ. ID. NO:1
[SEQ. ID. No:4]
   A base sequence of a DNA encoding the amino acid sequence represented by SEQ. ID. NO:2
[SEQ. ID. No:5]
   A base sequence of a synthetic primer used for cloning a DNA encoding a human lung-derived phosphodiesterase V of the invention in Example 1 described below
[SEQ. ID. No:6]
   A base sequence of a synthetic primer used for cloning a DNA encoding a human lung-derived phosphodiesterase V of the invention in Example 1 described below
[SEQ. ID. No:7]
   An amino acid sequence of a human lung-derived phosphodiesterase V of the invention
[SEQ. ID. No:8]
   An amino acid sequence corresponding to the deletion of 109 amino acids starting from the N-terminal of the amino acid sequence represented by SEQ. ID. NO:7
[SEQ. ID. No:9]
   A base sequence of a DNA encoding the amino acid sequence represented by SEQ. ID. NO:7
[SEQ. ID. No:10]
   A base sequence of a DNA encoding the amino acid sequence represented by SEQ. ID. NO:8

A transformant Escherichia coli BL21/pPDE51 obtained in Example 2 described below has been deposited to National Institute of Bioscience and Human Technology of Agency of Industrial Science and Technology of the Ministry of International Trade and Industry (NIBH) on July 13, 1998 under the deposition number FERM BP-6417 and also to Institute for Fermentation (IFO) on June 18, 1998 under the deposition No.IFO 16185.

### Example

The present invention is further detailed in the following Examples, which are not intended to restrict the invention. A gene engineering procedure employing E.coli was in accordance with the methods described in Molecular Cloning.

### Example 1 Cloning of gene encoding human lung-derived phosphodiesterase

CDNA was cloned using a Gene Trapper Positive Selection System (Gibco BRL).

An E.coli microorganism selected was subjected to a DNA extraction and reacted using a Thermo Sequenase Core Sequencing Kit (Amersham) to determine the base sequence of a cDNA fragment using an SQ-300 DNA Sequencer (HITACHI). The clone thus obtained had the 3036 base sequence comprising 2499 base sequence represented by SEQ. ID. No.9. This cDNA fragment encoded a novel phosphodiesterase V consisting of 833 amino acids represented by SEQ. ID. No.7. It had a 92 % homology with a known cattle-derived phosphodiesterase V (Linda M. McAllister et al., J.Biol.Chem. 268 (30), 22863 (1993), NCBI GenBank Accession No.L16545) at the level of the amino acids.

A plasmid pPDE50 having a DNA encoding a human lung-derived phosphodiesterase V of the invention was transduced into an E.coli DH10B to obtain a transformant E.coli DH10B/pPDE50.

### Example 2 Construction of E.coli expression vector

A cDNA encoding a human lung-derived phosphodiesterase V of the invention was digested with EcoRI and XhoI and ligated with a pGEX4T-2 (Pharmacia) which had similarly been treated. Using the ligation solution, an E.coli BL21 (FUNAKOSHI) was transformed to obtain an E.coli BL21/pPDE51 capable of expressing the human lung-derived phosphodiesterase V of the invention.

### Example 3 Expression of recombinant human lung-derived phosphodiesterase V in E.coli and purification

Using the E.coli BL21/pPDE51 obtained in Example 2, a recombinant human lung-derived phosphodiesterase V was obtained. An expression in E.coli and a purification were performed in accordance with the protocol attached to a GST Gene Fusion System (Pharmacia). As a result, 12.5 mg of an intended recombinant human lung-derived phosphodiesterase V of about 100 kDa was recovered from 1L of an E.coli culture.

### Example 4 Detection of human lung-derived phosphodiesterase V

The phosphodiesterase activity of a human lung-derived phosphodiesterase V was detected using a Phosphodiesterase [³H] cGMP SPA enzyme assay kit (Amersham). As a result, a phosphodiesterase activity was detected in the enzyme solution obtained in Example 2. A BL21 transformed with a pGEX4T-2 employed as a control exhibited no phosphodiesterase activity.

### Example 5 Establishment of inhibitor screening system

A 96-well microplate (OPTI Plate, Packard) received 10 µL of a buffer solution [0.5M Tris-HCl (pH7.5), 83 mM MgCl₂, 17 mM EGTA], 10 µL of the recombinant human lung-derived phosphodiesterase V obtained in Example 3 (0.025 mg/ml), 65 µL of an ultrapure water, 5 µL of an inhibitor sample and 10 µL of [³H]cGMP, and reacted at 30°C for 30 minutes. After completion of the reaction, 50 µL of a SPA beads solution [18 mg/ml Yttrium silicate beads, 18 mM ZnSO₄] was added and allowed to stand at room temperature for about 20 minutes, and then subjected to a scintillation counting (Topcount, Packard). The radioactivity in the presence of the recombinant human lung-derived phosphodiesterase V was 10367 cpm which was contrast to the radioactivity in the absence of the recombinant human lung-derived phosphodiesterase V (1780 cpm). In this reaction, the phosphodiesterase activity was inhibited by adding various concentration of a phosphodiesterase V inhibitor SHILDENAPHILE (Drugs of Future, 22 (2), 1997), and SHILDENAPHILE inhibited this enzyme reaction by 50% at about 2 nM. Thus, this assay system was proven to enable the screening for a phosphodiesterase inhibitor.

### Example 6 Screening for inhibitor

The method established in Example 5 was employed to screen for a recombinant human lung-derived phosphodiesterase inhibitor. As a result, some polysaccharides exhibited the inhibitory effects. An example of such polysaccharides is one represented by Formula 1 shown below. At 2.29 nM, this compound inhibited the enzyme reaction by 50 %.

### (Formula 1)

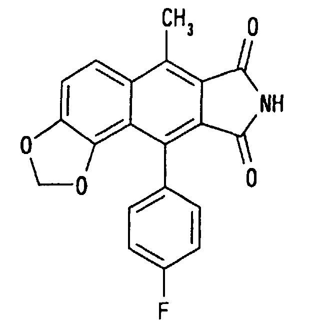

### Industrial Applicability

A protein of the invention is useful as an agent for screening for a compound which promotes or inhibits a function of the protein of the invention (typically a phosphodiesterase activity, more preferably a phosphodiesterase V activity, most preferably, a phosphodiesterase activity specific to a protein having the amino acid sequence represented by SEQ. ID. NO:7) or its salt. In addition, an antibody against a protein of the invention can recognize the protein of the invention specifically, and thus can be used, for example, for quantifying the protein of the invention in a test sample.

## Claims

1. A protein having an amino acid sequence identical or substantially identical to the amino acid sequence represented by SEQ. ID. NO:7 or its salt.

2. The protein according to Claim 1 wherein the substantially identical amino acid sequence is the amino acid sequence represented by SEQ. ID. NO:1.

3. The protein according to Claim 1 which is encoded by a cDNA possessed by an E.coli plasmid pPDE51 designated by Deposition No. FERM BP-6417.

4. The protein according to Claim 1 having a phosphodiesterase activity.

5. A partial peptide of the protein according to Claim 1 comprising the first to the tenth amino acid sequence from the N-terminal of the amino acid sequence represented by SEQ. ID. NO:7 or its salt.

6. A DNA comprising a DNA having a base sequence encoding the protein according to Claim 1 or the partial peptide according to Claim 5.

7. The DNA according to Claim 6 having the base sequence represented by SEQ. ID. NO:9.

8. A recombinant vector comprising the DNA according to Claim 6.

9. The recombinant vector according to Claim 8 which is an expression vector in E.coli.

10. A transformant possessing the recombinant vector according to Claim 8.

11. The transformant according to Claim 10 whose host cell is E.coli.

12. A method for producing the protein according to Claim 1 or its salt comprising incubating the transformant according to Claim 10 to produce and accumulate the protein according to Claim 1 followed by collecting this protein.

13. An antibody against the protein according to Claim 1, the partial peptide according to Claim 5 or its salt.

14. A method for screening for a compound or its salt which promotes or inhibits the phosphodiesterase activity of the protein according to Claim 1, the partial peptide according to Claim 5 or its salt, comprising using the protein according to Claim 1, the partial peptide according to Claim 5 or its salt.

15. A kit for screening for a compound or its salt which promotes or inhibits the phosphodiesterase activity of the protein according to Claim 1, the partial peptide according to Claim 5 or its salt, comprising using the protein according to Claim 1, the partial peptide according to Claim 5 or its salt.
